# EUROPEAN PATENT APPLICATION

(11) **EP 3 378 393 A1**
(43) Date of publication of application: **26.09.2018**
(21) Application number: 16866445.6
(22) Date of filing: 18.11.2016
(51) Int. Cl.: A61B 5/07, G01N 27/30, G01N 27/327

(54) **BIOSENSOR AND MANUFACTURING METHOD THEREFOR**

(30) Priority: 20.11.2015 JP 2015227645
(71) Applicant: Toppan Printing Co., Ltd., Tokyo 110-0016 (JP)
(72) Inventor: OKUBO, Toshikazu, Tokyo 110-0016 (JP); WARIGAYA, Ryo, Tokyo 110-0016 (JP); KOSUGI, Masahiro, Tokyo 110-0016 (JP); MINOWA, Kazuyo, Tokyo 110-0016 (JP)
(74) Representative: Cabinet Plasseraud
(86) International application number: PCT/JP2016/084286
(87) International publication number: WO 2017/086445

(57) **Abstract**

A highly reliable biosensor is provided. The biosensor of the present invention includes two or more electrodes (2a,2b,2c), and a support (3) supporting the two or more electrodes (2a,2b,2c). One or more of the electrodes (2a,2b) each include a substrate (21) having a metal surface, and a protective layer (22) covering at least part of the surface. The protective layer (22) is a continuous film made of carbon and having a thickness of at least 1 µm.

## Description

### [Technical Field]

The present invention relates to a biosensing technology.

### [Background Art]

Recently, medical treatments have developed to a high degree, and analyses of the patient's body fluids such as blood, saliva, and urine, can ascertain the state of health of the patient. For example, research is being conducted for judging the presence or absence of dental decay by measuring the pH of saliva, or for diagnosing diabetes by measuring the glucose level of tears. For example, examinations for these judgements are conducted by the patients themselves, collecting their body fluids, and then by medical institutions measuring and analyzing the collected body fluids.

On the other hand, developments are also being made for devices by which patients can measure and analyze their body fluids by themselves, obviating the need for the patients to go to medical institutions. These devices not only expedite the examinations and analyses but also contribute to reducing the medical cost in an aging society as described below.

Generally, after a patient recognizes that her/his health condition is poor, she/he will consult a physician at a medical institution. However, at that stage, the patient may be terminally ill. In this case, the patient may receive advanced medical treatments and administration of expensive medicines, and as a result, the burden of medical expenses increases.

If the poor health condition of the patient can be discovered earlier, the patient may be cured by reviewing the lifestyle of the patient, while receiving a mild treatment that does not require medication and the like. Therefore, there are an increasing number of institutions that conduct periodic health examinations as a preventive therapy organized by health insurance associations.

However, since the periodic medical examinations are generally conducted once or twice a year, there is a blank period between the examinations. Accordingly, a disease that has developed during this period cannot be recognized. In this way, the current preventive medical treatment has limitations.

If a patient can use a device with which the patient can measure and analyze the body fluids without having to go to a medical institution, examinations can be conducted at a high frequency. Therefore, the patient can discover changes in her/his physical condition before the patient becomes aware of the changes. Therefore, use of such devices decreases the necessities to patients of having to receive advanced medical treatments and administration of expensive medicines, resulting in reducing medical costs.

Methods of obtaining information on body fluids include a method of inserting a biosensor into a living body or affixing a biosensor to the skin or mucous membranes of a living body to acquire in vivo information of the living body, and a method of acquiring information of fluids collected from a living body in an ex vivo manner using a biosensor external to the living body. These methods of measuring and analyzing the body fluids by the patients themselves have advantages and disadvantages. For example, in the in vivo method, the information of the living body can be collected without a time lag. However, since the sensor is brought into direct contact with the living body, the influence of the sensor on the living body is great and high reliability is required of the device. In the ex vivo method, since the sensor is not brought into contact with the living body, the influence of the sensor on the living body is small. However, collection is difficult depending on the body fluids, and in addition, there is a concern that the collected body fluids will change over time. Depending on the usage and the purpose, an appropriate method is required to be selected.

There are various biomedical sensing methods. For measurement of blood sugar level and the like, electrochemical methods are widely used for the reason that these methods facilitate highly sensitive detection of trace components. In such an electrochemical method, bio-information corresponding to chemical properties are detected as electrical signals. Accordingly, there is an advantage that the obtained signals can be easily processed and analyzed using a semiconductor device or the like. Therefore, active development is globally underway for new electrochemical sensing devices and sensing methods using the devices.

For example, the following PTLs 1 to 5 describe technology relating to an ex vivo electrochemical sensing device.

JP 2012-101092 A (PTL 1) describes a method of measuring the concentration of an analyte component in a body fluid using an analyte sensor. The analyte sensor includes a sample chamber with a volume of no more than 1 µL. The chamber includes a working electrode, a counter/reference electrode, a redox mediator and an analyte reactive enzyme. The analyte sensor is an in vitro (synonymous with ex vivo) sensor in which the working electrode is apart from the counter/reference electrode by a distance of 200 µm or less.

JP H11-271259 A (PTL2) describes a technique of simplifying the manufacturing process and reducing the manufacturing cost of a sensor cartridge used for measuring urine sugar. The sensor cartridge includes a urine sugar sensor which is incorporated in the cartridge body.

JP 2002-207037 A (PTL 3) describes a technique of measuring an oxidation-reduction potential of saliva to judge physical conditions.

JP 2007-3256 A (PTL 4) describes a method of measuring a renal function control status. This method includes obtaining the concentrations of phosphoric acid and calcium in the blood separated from a living body through a single measurement using a measuring system, visibly displaying the measurement results, and judging the control status of the renal function in the living body on the basis of the displayed measurement results.

JP H6-148124 A (PTL 5) describes a disposable ion sensor unit. The ion sensor unit is configured to separate a channel through an ion sensor from a channel through a reference electrode. A liquid to be measured is supplied only into the channel through the ion sensor. An electrolyte containing chloride ions of a predetermined concentration is supplied into the channel through the reference electrode. These channels are connected to each other at the downstream thereof to form a liquid-liquid junction.

In these techniques, a body fluid such as blood, urine or saliva is collected to electrochemically measure blood sugar, urine sugar, activity ratio of oxidants/reductants, or ion (chlorine) concentration therein. In electrochemical sensing, chemical stability of the electrode material is important because the electrodes are brought into contact with the body fluid that is an analyte component.

As such an electrode material, carbon may be used besides precious metals. This is because carbon is an electrochemically inert material.

For example, WO 2010/004690 A (PTL 6) describes that carbon is used as an electrode material for an electrochemical sensor. The carbon electrode described in this patent document includes an insulating substrate, an electrically conductive layer provided on the insulating substrate, a first carbon layer provided on the conductive layer, and a second carbon layer covering the first carbon layer. The first carbon layer has sp2 bonds and sp3 bonds, and contains carbon having an amorphous structure. The second carbon layer contains carbon having SP2 bonds. The first carbon layer is specifically made of diamond-like carbon or amorphous carbon having an amorphous structure formed by vapor deposition.

WO 1999/045387 A (PTL 7) describes an electrochemical sensor for measuring the level of an analyte component, such as glucose, lactate, or oxygen, in vivo and/or ex vivo.

WO 2013/016573 A (PTL 8) describes a sensor suitable for being embedded in both solid and gel-like tissues, for in vivo detection and measurement. This sensor enables long term monitoring of glucose levels on a near-continuous or semi-continuous basis by wireless telemetry. This sensor includes a) a hermetically sealed housing, b) a detector array, c) an electrical power source such as a battery, d) circuitry having a function of accurately processing detector signals and operatively connected to the detector array, and e) a telemetry transmission portal including a means for stably transmitting processed detector signals to the exterior of the sensor to relay them to a receiver outside the body.

### [Summary of Invention]

The present invention has an object of providing a biosensor having high reliability.

According to a first aspect of the present invention, a biosensor is provided which includes two or more electrodes and a support supporting the two or more electrodes. In the biosensor, one or more of the electrodes each include a substrate having a metal surface, and a protective layer covering at least part of the surface; and the protective layer is a continuous film made of carbon and having a thickness of 1 µm or more.

According to a second aspect of the present invention, a manufacturing method for a biosensor is provided which includes a step of obtaining an electrode by forming a carbon protective layer using plating on a substrate having a metal surface so as to cover at least part of the surface and to have a thickness of at least 1 µm, and a step of allowing a support to support two or more electrodes including the electrode.

### [Brief Description of the Drawings]

Fig. 1 is a schematic perspective view illustrating a biosensor, according to a first embodiment of the present invention.
Fig. 2 is a block diagram illustrating the configuration of the biosensor illustrated in Fig. 1.
Fig. 3 is a schematic diagram illustrating a usage example of the biosensor illustrated in Fig. 1.
Fig. 4 is a schematic perspective view illustrating a biosensor according to a second embodiment of the present invention.
Fig. 5 is a schematic perspective view illustrating a biosensor according to a third embodiment of the present invention.
Fig. 6 is diagram illustrating a cross-section of part of the biosensor illustrated in Fig. 5.
Fig. 7A is a plan view illustrating a first process in the manufacture of the biosensor illustrated in Figs. 5 and 6.
Fig. 7B is a cross-sectional view taken along the line VIIb-VIIb in the structure illustrated in Fig. 7A.
Fig. 8A is a plan view illustrating a second process in the manufacture of the biosensor illustrated in Figs. 5 and 6.
Fig. 8B is a cross-sectional view taken along the line VIIIb-VIIIb in the structure illustrated in Fig. 8A.
Fig. 9A is a plan view illustrating a third process in the manufacture of the biosensor illustrated in Figs. 5 and 6.
Fig. 9B is a cross-sectional view taken along the line IXb-IXb in the structure illustrated in Fig. 9A.
Fig. 10A is a plan view illustrating a fourth process in the manufacture of the biosensor illustrated in Figs. 5 and 6.
Fig. 10B is a cross-sectional view taken along the line Xb-Xb in the structure illustrated in Fig. 10A.
Fig. 11A is a plan view illustrating a fifth process in the manufacture of the biosensor illustrated in Figs. 5 and 6.
Fig. 11B is a cross-sectional view taken along the line XIb-XIb in the structure illustrated in Fig. 11A.
Fig. 12A is a plan view illustrating a sixth process in the manufacture of the biosensor illustrated in Figs. 5 and 6.
Fig. 12B is a cross-sectional view taken along the line XIIb-XIIb in the structure illustrated in Fig. 12A.
Fig. 13A is a plan view illustrating a seventh process in the manufacture of the biosensor illustrated in Figs. 5 and 6.
Fig. 13B is a cross-sectional view taken along the line XIIIb-XIIIb in the structure illustrated in Fig. 13A.
Fig. 14 is a plan view illustrating a first process in the manufacture of a biosensor according to a fourth embodiment of the present invention.
Fig. 15 is a cross-sectional view taken along the line XV-XV in the structure illustrated in Fig. 14.
Fig. 16 is a schematic perspective view illustrating an electrode obtained by performing a second process in the manufacture of the biosensor according to the fourth embodiment.
Fig. 17 is a cross-sectional view illustrating a third process in the manufacture of the biosensor according to the fourth embodiment.
Fig. 18 is a cross-sectional view illustrating a fourth process in the manufacture of the biosensor according to the fourth embodiment.
Fig. 19 is a schematic diagram illustrating the biosensor according to the fourth embodiment, with part being a cross-sectional view, and the rest being a perspective view.
Fig. 20 is a schematic perspective view illustrating a first process in a connection method according to a modification.
Fig. 21 is a schematic perspective view illustrating a second process in the connection method according to the modification.
Fig. 22 is a schematic perspective view illustrating a connection method according to another modification.
Fig. 23 is a schematic diagram illustrating a biosensor according to a fifth embodiment the present invention.
Fig. 24 is a schematic diagram illustrating a modification of the biosensor illustrated in Fig. 23.
Fig. 25 is a schematic diagram illustrating another modification of the biosensor illustrated in Fig. 23.
Fig. 26 is a schematic diagram illustrating still another modification of the biosensor illustrated in Fig. 23.
Fig. 27 is a schematic diagram illustrating a biosensor according to a sixth embodiment the present invention.
Fig. 28 is a schematic diagram illustrating a modification of the biosensor illustrated in Fig. 27.
Fig. 29 is a schematic diagram illustrating another modification of the biosensor illustrated in Fig. 27.
Fig. 30 is a schematic plan view illustrating a first process of a method available for the manufacture of electrodes provided to the biosensor illustrated in Figs. 23 and 27.
Fig. 31 is a plan view illustrating the structure illustrated in Fig. 30, as viewed from the back.
Fig. 32A is a cross-sectional view taken along the line XXXIIa-XXXIIa in the structure illustrated in Fig. 30.
Fig. 32B is a cross-sectional view taken along the line XXXIIb-XXXIIb in the structure illustrated in Fig. 30.
Fig. 32C is a cross-sectional view taken along the line XXXIIc-XXXIIc in the structure illustrated in Fig. 30.
Fig. 33A is a schematic cross-sectional view, corresponding to Fig. 32A, illustrating a second process of a method available for the manufacture of the electrodes provided to the biosensor illustrated in Figs. 23 and 27.
Fig. 33B is a schematic cross-sectional view, corresponding to Fig. 32B, illustrating a second process of a method available for the manufacture of the electrodes provided to the biosensor illustrated in Figs. 23 and 27.
Fig. 33C is a schematic cross-sectional view, corresponding to Fig. 32C, illustrating a second process of a method available for the manufacture of the electrodes provided to the biosensor illustrated in Figs. 23 and 27.
Fig. 34A is a schematic cross-sectional view, corresponding to Fig. 32A, illustrating a third process of a method available for the manufacture of the electrodes provided to the biosensor illustrated in Figs. 23 and 27.
Fig. 34B is a schematic cross-sectional view, corresponding to Fig. 32B, illustrating a third process of a method available for the manufacture of the electrodes provided to the biosensor illustrated in Figs. 23 and 27.
Fig. 34C is a schematic cross-sectional view, corresponding to Fig. 32C, illustrating a third process of a method available for the manufacture of the electrodes provided to the biosensor illustrated in Figs. 23 and 27.
Fig. 35 is a schematic plan view illustrating a third process of a method available for the manufacture of the electrodes provided to the biosensor illustrated in Figs. 23 and 27, that is, illustrating a structure obtained from the third process, as viewed from the back.
Fig. 36 is a schematic plan view illustrating a fourth process of a method available for the manufacture of the electrodes provided to the biosensor illustrated in Figs. 23 and 27, that is, illustrating a structure obtained from the fourth process, as viewed from the back.
Fig. 37 is a schematic side view illustrating a first example of the biosensor according to a seventh embodiment of the present invention.
Fig. 38 is a schematic side view illustrating a second example of the biosensor according to the seventh embodiment of the present invention.
Fig. 39 is a schematic side view illustrating a third example of the biosensor according to the seventh embodiment of the present invention.
Fig. 40 is a schematic diagram illustrating a usage example of the biosensor according to the seventh embodiment of the present invention.
Fig. 41 is a schematic diagram illustrating another usage example of the biosensor according to the seventh embodiment of the present invention.
Fig. 42 is a schematic plan view illustrating a process in the manufacture of the biosensor according to Example 3 that is a specific example of the present invention.
Fig. 43 is a cross-sectional view taken along the line XLIII-XLIII in the structure illustrated in Fig. 42.
Fig. 44 is a schematic perspective view illustrating a process in the manufacture of the biosensor according to Example 3 that is a specific example of the present invention.

### [Description of the Embodiments]

The embodiments of the present invention will be described below.

### <Description of Prior Art>

Issues found by the present inventors regarding the conventional art in inventing the present invention will be described below.

As shown in PTLs 1 to 8, several inventions have been made for biosensors for electrochemically measuring and analyzing the components of a body fluid obtained from a living body to obtain numerical values as biomarkers. The biosensors described in PTLs 1 to 5 use an ex vivo method, with the forms and processes being adapted to the object measurement system. The biosensor described in PTL 6 uses carbon as the material for the electrodes for electrochemical measurement. The biosensors described in PTLs 7 and 8 each include a control device/processor and an element that transmits signals.

Such existing biosensors need to improve reliability of the devices. To further promote the widespread use of these biosensors, it is desirable to reduce cost and improve productivity in manufacture.

One of the most important points in realizing these matters resides in the electrodes which are the most important element when performing electrochemical measurements. Specifically, the point is whether such electrodes are suitable for the actual manufacture, in terms of the form, material, the manufacturing method, and any other matters including cost.

Regarding the form of the electrodes, it is necessary that the measurement electrodes can have any shape suitable for the application. For example, being imparted with a curved surface rather than a flat surface, the measurement electrodes will have improved compatibility with a diversity of devices.

The electrode surfaces must be electrochemically stable. For example, each electrode made of conducting base material may be partially coated with an electrochemically inert conductive material and covered with an insulating support material at the portion not coated with the conductive material. When the electrode of such a structure is used and if liquid permeates between the insulating material and the electrode, the conductive material, which is electrochemically unstable, contacts the liquid, resulting in corrosion and insufficient measurement reliability. Further, even if liquid is prevented from permeating between the insulating material and the electrode, measurement still cannot be made with sufficient reliability if the coating of the conductive material is so thin that generation of pinholes cannot be prevented at the manufacturing stage.

Problems such as corrosion do not occur if the electrochemically inert conductive material is applied onto a non-conductive material. However, in this case, unless the coating of the conductive material is formed considerably thick, a large current cannot be supplied in a range ensuring accuracy in amperometric or coulometric measurement.

Generally, precious metal such as gold or platinum is selected as the electrochemically stable conductive material. Such a substance is remarkably costly, and thus, is formed as a thin film on a substrate, and made into the shape of an electrode by patterning. However, as the film needs to have a thickness ensuring prevention of pinholes, the cost will be high even if the film is thin.

In this regard, as described in PTL 6, carbon may be used as a material for such an electrode because it is electrochemically stable and low cost. However, carbon is a material that is very difficult to process and cannot form a thick and dense layer with vapor deposition or printing. Therefore, carbon has not substantially been considered as a material for use in the electrode of a biosensor.

The general manufacturing methods of electrodes, i.e. conductors, can be classified into subtractive methods and additive methods. Examples of the subtractive methods include (1) a method to form a conductive material into an electrode shape by applying physical processing, such as cutting, polishing, molding or laser processing, to a single piece of a plate-, linear-, or lump-shaped conductive material, or by applying chemical processing, such as chemical etching, to the single piece of conductive material, and (2) a method to form a conductive material into an electrode shape by forming a layer of the conductive material on an insulating substrate with a comparatively large area, and by applying physical or chemical processing to the layer. Examples of the additive methods include (3) a method to form a conductive material into an electrode shape by forming a layer of the conductive material on an insulating substrate such as by printing, and (4) a method to form an electrode through consecutive steps of forming a thin layer of a conductive material on an insulating substrate, covering this thin layer with a partially opened mask, thickly depositing an electrode material into the openings, and removing the mask and the conductive film under the mask.

The electrodes obtained by the methods (1) to (4) may be partially coated with an insulating material such as a resin. A different metal may be deposited on the electrode surfaces by a film forming method such as plating or vapor deposition.

The methods (1) to (4) are useful in the manufacture of electrodes having metal surfaces. However, as will be described below, there are many restrictions in the manufacture of carbon electrodes using these methods.

When obtaining carbon electrodes through the method (1), chemical processing cannot substantially be used. To obtain carbon electrodes through the method (1), a carbon material, such as glassy carbon, boron doped diamond or graphite, is physically processed. Therefore, it is difficult for this kind of method to fabricate fine electrodes and to improve productivity through collective processing of the electrodes.

To obtain carbon electrodes through the method (2), conductive carbon, such as graphite, graphene or carbon nanotube, is deposited on the entire surface of an insulating substrate by vapor deposition, and the necessary portions of the obtained layer are covered with a mask, followed by dry etching of the portions not covered with the mask. However, it is difficult to increase the thickness of the carbon layer formed by vapor deposition.

To obtain carbon electrodes through the method (3), a carbon paste is printed. Alternatively, a mask having openings is formed, followed by vapor-depositing carbon nanotube, graphene or the like into the openings. However, the electrodes formed by printing a carbon paste entail formation of gaps between the carbon particles, which leads to impairing conductivity and allowing permeation of liquid into the electrode material. Accordingly, this method is not suitable for applications having a high reliability.

To obtain carbon electrodes through the method (4), vapor deposition is used in the same manner as in the method (2). Therefore, as previously stated, it is difficult to increase the thickness of the carbon layer.

According to PTL 6, the biosensor has a multilayer structure of insulating substrate / conductive layer / first carbon layer / second carbon layer, in which conductive layer / carbon layer are provided on a flat insulating substrate. Therefore, when used as electrodes, complicated process, such as dry etching or lift-off using a resist, has to be performed to pattern the structure into an electrode shape. Further, vapor deposition is not a practical method for forming a carbon layer with a sufficient thickness (e.g., 1 µm or more). It is true that vapor deposition can form a film of a uniform thickness on a flat substrate; however, it is difficult to form a carbon layer of a uniform thickness on a substrate deformed in advance, for example, and having a complicated shape.

Besides vapor deposition, methods of forming a carbon layer include printing a carbon paste, polishing glassy carbon or diamond, and applying a mixture of a carbon material and a polymer. However, it is difficult to form electrodes having a complicated shape with any of these methods. Since biosensors are restricted in the electrode shape for various reasons, methods enabling formation of carbon electrodes with a complicated shape have been potentially sought.

When attempting to use electrochemically stable and inexpensive carbon electrodes in the electrochemical sensor including the control device/processor as shown in PTLs 7 and 8, it is considered that the electrical contribution of the resistance, capacity, and inductor components of the carbon electrodes and electrode portions in the vicinity thereof may affect the balance of the entire device. Therefore, in this kind of system, it is necessary that each carbon electrode portion and the connection portion thereof have a comparatively high conductivity, and do not contain unwanted capacitance or unwanted inductance components. In the electrodes fabricated by printing a carbon paste, the resistance becomes large and the compatibility is poor, specifically in the case of fine wiring.

As stated above, existing electrochemical sensors have many problems in keeping up with the progress of medical technology in the future, improving reliability as devices, reducing costs and improving productivity for further promoting the widespread use of the sensors.

### <Embodiments>

One or more problems set forth above may be solved by biosensors according to embodiments.

A biosensor according to an embodiment uses, for example, a liquid collected from a living body or a liquid containing minute organisms, as a liquid sample, to electrochemically acquire numerical values ex vivo for serving as indexes for ascertaining the characteristics and states of the living body, and electrically transmit the numerical values to the outside. Alternatively, the biosensor described below uses a liquid of a living body as a liquid sample to electrochemically acquire numerical values in vivo for serving as indexes for ascertaining the characteristics and states of the living body, and electrically and externally transmit the numerical values.

To electrochemically acquire information, two or more electrodes are used. For example, the two or more electrodes are any of: one or more pairs of measurement electrodes; a pair of a measurement electrode and a reference electrode; and a combination of one or more pairs of measurement electrodes with a reference electrode.

At the time of measurement, these electrodes are brought into contact with the sample. The sample is, for example, a liquid sample. Examples of the liquid sample include blood, urine, sweat, tear, saliva, sebum, lymph, gastric juice, feces, semen, and nasal mucous, and dilutions of these in a liquid such as water.

When the two or more electrodes are one or more pairs of the measurement electrodes, a voltage is applied, or a current is passed across the paired measurement electrodes, and the current or the potential corresponding to the voltage or the current is measured. When measuring the current, if the measurement is continuously performed, the electrical charge can be obtained by integrating the current with time.

When the two or more electrodes are a pair of a measurement electrode and a reference electrode, the potential difference between the measurement electrode and the reference electrode is measured.

When the two or more electrodes are a combination of one or more pairs of measurement electrodes with a reference electrode, the potential difference between the reference electrode and one of paired measurement electrodes is kept constant, and the current flowing across the measurement electrodes of the pair is measured. Alternatively, a constant current is passed across paired measurement electrodes, and the potential difference between the reference electrode and one of measurement electrodes of the pair is measured.

Based on the measurement values obtained in this manner, a numerical value serving as a biomarker, such as a concentration, of the liquid sample is calculated.

The biosensor according to the embodiment uses a structure including a substrate having electrodes, one or more of which each have a metal surface, and a protective layer covering at least part of the surface. The protective layer is a continuous carbon film with a thickness of 1 µm or more.

Carbon is electrochemically inert. Therefore, this protective layer is hardly deteriorated, if it is brought into contact with a living body or a liquid.

Since carbon is electrochemically inert, it is stable in a living body. Therefore, when this protective layer is brought into contact with a living body, the influence on the living body is small.

As stated above, this protective layer is a continuous film. Namely, this protective layer is not porous or not formed of carbon particles but is a densely formed film. This is what is different from the protective layer formed of a carbon paste. This protective, which is sufficiently thick, has a low probability of causing pinholes. Therefore, the electrode is quite unlikely to allow a liquid to permeate the protective layer and contact the substrate.

As stated above, this protective layer is not porous or not formed of carbon particles but is a continuous film. Therefore, this protective layer has a high conductivity.

Accordingly, this biosensor is superior in reliability, and permits measurement with high precision.

Carbon is an inexpensive material compared to precious metals, and price fluctuations are small. Therefore, this biosensor can be manufactured at comparatively low cost.

As will be clear from the above description, it is preferred that the protective layer fully covers the region in each surface with which the measuring object at least contacts.

Since the protective layer is made of carbon, it is difficult to bond the lead wires thereto by soldering or the like. When a lead wire is bonded to an electrode, for example, the metal surface of the substrate is partially exposed in advance, and the lead wire is bonded to the substrate at the exposed position.

For example, first, a protective layer is formed on the entirety of the metal surface of the substrate. Then, the protective layer is partially removed. Then, an end of a lead wire is bonded to the exposed portion of the metal surface of the substrate.

Alternatively, a protective layer is formed on the entire surface of a substrate made of a metal, and then, an edge of the substrate is cut together with the protective layer on top thereof. Thus, the metal is exposed at the position of the cut surface. For example, a plate having a metal surface is partially removed to obtain a structure that includes a substrate and a support part connected thereto. Then, a protective layer is formed on the metal surface of the structure. After that, the substrate is cut off from the support part. Then, an end of a lead wire is bonded to the exposed portion of the metal surface of the substrate.

Alternatively, first, a mask is formed in part the metal surface of a substrate. Then, a protective layer is formed on the entirety of the exposed part of the metal surface. After that, the mask is removed from the metal surface. Then, an end of a lead wire is bonded to the exposed portion of the metal surface of the substrate.

In any one of the methods, the protective layer preferably covers the entirety of the aforementioned surface except for the joint between the metal surface of the substrate and the lead wire.

The substrate may have various shapes. When the substrate has a plate-like shape, two or more electrodes may be arranged parallel to each other or may be arranged so as to be inclined against each other. Further, the substrate may have, for example, a coil shape or a leaf-spring shape. It should be noted that when an electrode has a coil shape, the electrode may also serve as a spring.

The electrodes and the respective lead wires may be electrically connected using the following methods. First, apertures are provided to a metal surface of a substrate. Then, a protective layer is formed on the entirety of the exposed part of the metal surface. Then, a pin or a screw is inserted into each aperture so that a lead wire connected to the pin or the screw can be electrically connected to the electrode.

When an electrode is formed by providing through holes to a substrate and forming a protective layer on the entirety of the exposed part of the metal surface, a pin, a screw or the like may be inserted into each through hole, for example, to secure the electrode to a separately prepared substrate, such as a substrate having a metal surface, while establishing electrical connection between this substrate and the electrode. Thus, the electrodes can be detached from the sensor body, or may be attached thereto, as necessary. Accordingly, for example, only the electrodes can be disposed. The aforementioned protective layer may also be formed on the surface of the separately prepared substrate.

Since this method forms a protective layer made of carbon by plating, the protective layer can be uniformly formed even on the sidewalls of the respective apertures. Namely, this method has an advantage of not allowing the metal surface to be exposed.

The biosensor may further include a processing unit (or an information processing unit) which is electrically connected to two or more electrodes to generate information relating to the measuring object, based on the current or voltage across the electrodes. In this case, the biosensor may further include an output unit for outputting information to the outside of the biosensor. Further, the biosensor may also include a power supply unit and a control unit.

For example, the aforementioned protective layer is formed by plating. For example, the plating may be the carbon plating technique using molten salt electrolysis, which has been put into practice by I'MSEP Co., Ltd. This technique forms a very dense carbon layer on a surface of a material, i.e. an anode, to be treated, by using an anodizing reaction of carbide ions (C₂²⁻) added to a molten salt such as chloride (see the reaction formula below).

C₂²⁻→ 2C (plating film) + 2e⁻

The protective layer obtained in this manner typically includes a graphite structure, and is made of a mixture of sp2-bonded carbon atoms and sp3-bonded carbon atoms. The details of the method are described in JP 2009-120860 A.

In another method using molten salt electrolysis to obtain a carbon protective film, carbonate is added to a molten salt to reduce the material to be treated as a cathode. This reaction is expressed by the following reaction formula.

CO₃²⁻ + 4e⁻ →C + 3O²⁻

The details of this method are described in JP 2006-169554 A.

There is another method using an electrochemical reaction for formation of a carbon protective layer on a surface of a substrate. In this method, an electrolyte containing a metal to be deposited is permitted to contain some conductive carbon particles to induce an electrochemical reaction such that the substrate becomes a cathode to thereby form a protective layer.

For example, the carbon particles to be used may have a graphite structure and made up of a mixture of sp2 structure and sp3 structure.

The metal to be deposited may be appropriately selected to be one which can be used in an electroplating solution in the form of an aqueous solution. Besides precious metals such as gold, platinum, silver, rhodium and ruthenium, examples of the metal to be deposited include iron, nickel, cobalt, copper, chromium, zinc, or alloys of these metals. Alternatively, when a non-aqueous dimethyl sulfone bath is used, aluminum may be used as the metal to be deposited.

A precious metal is preferably used if the liquid sample as an object of biomedical sensing contains a comparatively high concentration of a compound which is acidic or alkaline or which can form a complex with the metal to be deposited. If the liquid sample is neutral and does not contain a compound which can form a complex with the metal to be deposited, there may be variety of choices of metals. Usability as a metal to be deposited may be verified by experiment in advance.

The protective layer obtained by these methods is not a pure carbon layer because, strictly speaking, the metal to be deposited is deposited around the carbon particles. However, the protective layer can be used by selecting the metal to be deposited according to the object of biomedical sensing.

As stated above, the protective layer is formed such as by plating. Plating can form a thick protective layer. For example, a protective layer with a thickness of 1 to 5 µm can be formed.

Further, plating can form a protective layer with a uniform thickness, even when the substrate has a complicated shape. For example, when the substrate has a curved or bent shape, a protective layer can be formed covering at least the region in the metal surface of the substrate, corresponding to the curved or bent portion of the substrate. When a substrate has first and second main surfaces parallel to each other, and an edge face extending along the edges thereof, a protective layer may be formed covering the entirety of the first main surface, at least part of the second main surface, and at least part of edge face.

Further, plating can form a protective layer at comparatively low cost and with high productivity.

According to plating, a protective layer can be easily formed on the entire surface of a substrate.

Therefore, this technique can provide a biosensor that can follow up the advancement of medical technology in the future, improve reliability as a device, and achieve low cost and improvement in productivity.

When a carbon protective layer is formed by plating, the protective layer should typically have a graphite structure and be made of a mixture of sp2-bonded carbon atoms and sp3-bonded carbon atoms.

The surface of the protective layer may be modified to improve sensitivity in biomedical sensing measurement. For example, when blood sugar is analyzed, glucose oxidase or an osmium compound may be immobilized on the surface of the protective layer.

With reference to the drawings, the embodiments will be described hereinbelow. To omit duplication, the components exerting identical or similar functions are given the same reference numerals in the accompanying drawings.

### <First Embodiment>

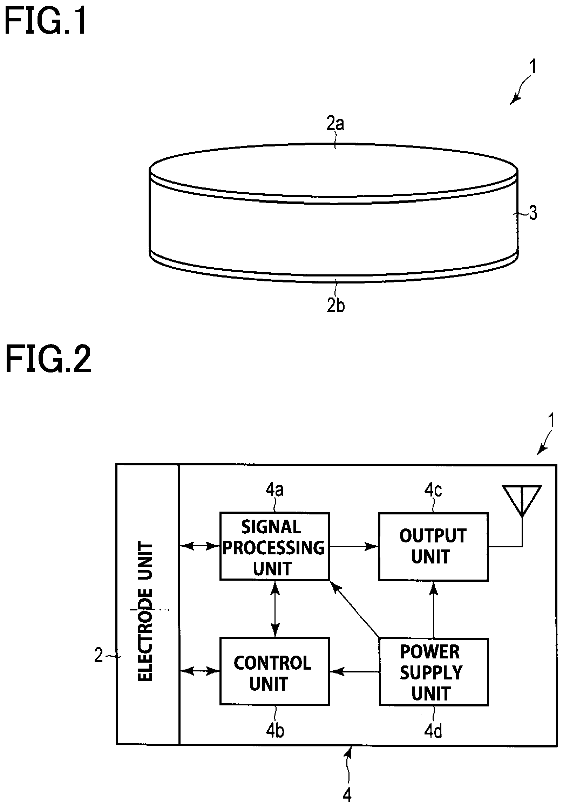

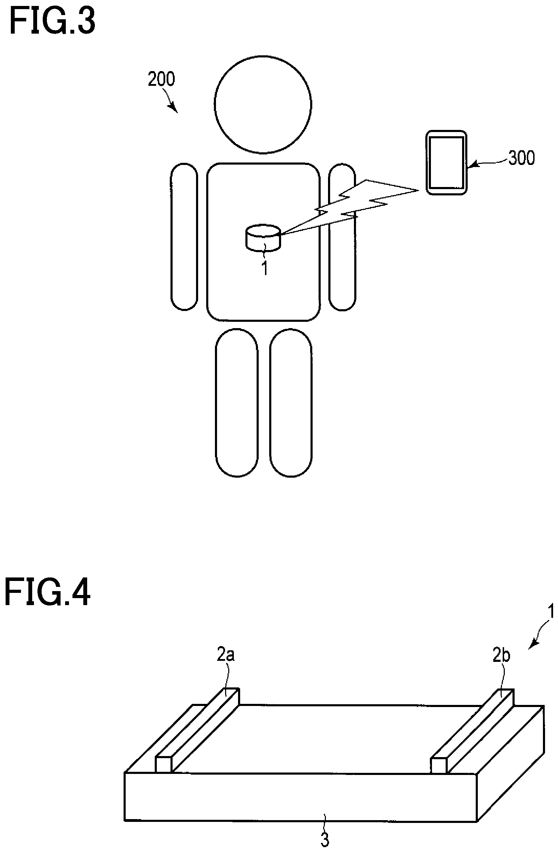

Fig. 1 is a schematic perspective view illustrating a biosensor according to a first embodiment of the present invention. Fig. 2 is a block diagram illustrating a configuration of the biosensor shown in Fig. 1. Fig. 3 is a schematic diagram illustrating a usage example of the biosensor shown in Fig. 1.

As shown in Fig. 3, the biosensor 1 shown in Figs. 1 and 2 is swallowed by a living body 200 that is a person herein to obtain information relating to the living body 200 from the fluids of the living body 200. The biosensor 1 transmits this information wirelessly to an external device 300. Therefore, the information relating to the living body 200 can be collected, for example, in real time. The information relating to the living body 200 may be stored in the biosensor 1, and after the biosensor 1 has been excreted from the living body 200, the information may be transmitted from the biosensor 1 to the external device 300 in a wireless or wired fashion.

The biosensor 1 has a flat and substantially columnar shape similar to a generally used tablet, but the shape of the biosensor 1 is not limited to this. For example, the biosensor 1 may have a flat elliptical columnar shape or an elongated columnar shape, or may have a spherical or a spheroidal shape.

The biosensor 1 includes an electrode unit 2 and an electronic component 4 shown in Fig. 2, a support 3 shown in Fig. 1, and a battery, not shown.

The electrode unit 2 shown in Fig. 2 includes the electrodes 2a and 2b shown in Fig. 1. One or both of the electrodes 2a and 2b include a substrate and a protective layer. As an example herein, both of the electrodes 2a and 2b include a substrate and a protective layer described below.

The substrate has a metal surface. For example, the substrate is made of a metal, or is a composite of a metal and an insulator, such as a resin. The metal that can be used may be stainless steel or iron.

In the structure shown in Fig. 1, each substrate has first and second main surfaces parallel to each other and an edge face extending along the edges of the first and second main surfaces. Herein, the substrate is disc-shaped.

The protective layer covers at least part of the metal surface of the substrate. The protective layer is the aforementioned continuous film made of carbon. As an example herein, the protective layer covers the entirety of the first main surface of the substrate, part of the second main surface, and the entirety of the edge face.

The support 3 supports the electrodes 2a and 2b so that they are separated from each other. At least a portion of the support 3 contacting the electrodes 2a and 2b is formed of an insulator. For example, a medical plastic or ceramic can be used as the insulator.

In the structure shown in Fig. 1, the support 3 has a flat cylindrical shape. The support 3 supports the electrodes 2a and 2b, with one bottom surface thereof facing the second main surface of the electrode 2a, and the other bottom surface thereof facing the second main surface of the electrode 2b.

The support 3 has a hollow structure. The support 3 is internally provided with the electronic component 4 shown in Fig. 2, and a battery, not shown.

The electronic component 4 shown in Fig. 2 includes a signal processing unit (or a processing unit) 4a, a control unit 4b, an output unit 4c, and a power supply unit 4d.

The signal processing unit 4a and the control unit 4b are electrically connected to the electrode unit 2. The control unit 4b applies a voltage or a current across the electrodes 2a and 2b. The signal processing unit 4a generates information relating to the living body 200 from the magnitude of the current flowing across the electrodes 2a and 2b or the potential difference therebetween.

The magnitude of the voltage applied across the electrodes 2a and 2b, or the current supplied therebetween by the control unit 4b may be, for example, constant, or may be changed to pulse waves, or may be linearly increased or decreased with time, or may be changed by a function with time.

The signal processing unit 4a, for example, converts the magnitude of the current flowing across the electrodes 2a and 2b or the potential difference therebetween to a signal which is easily transmitted to the output unit 4c. The signal processing unit 4a may further perform averaging or model-based calculations or the like.

The power supply unit 4d is electrically connected to the signal processing unit 4a, the control unit 4b, and the output unit 4c. The power supply unit 4d appropriately distributes the power supplied from the battery to other elements of the electronic component 4. Alternatively, the power supply unit 4d may include a battery.

The output unit 4c is electrically connected to the signal processing unit 4a. The output unit 4c uses wireless communication to transmit the information generated by the signal processing unit 4a to the external device 300. Use of wireless communication not only reduces the risk of malfunction due to the mixing of foreign matter into the inside of the biosensor 1, but also provides hygienic advantages.

The signal processing unit 4a, the control unit 4b, the output unit 4c, and the power supply unit 4d are formed on one or more semiconductor chips. All of these units may be formed on a single semiconductor chip, or these units may be formed on separate semiconductor chips.

The battery, not shown, is electrically connected to the power supply unit 4d. The battery supplies electric power to the power supply unit 4d. The biosensor 1 does not have to include a battery if, for example, power can be supplied from outside in a wireless manner, or if the biosensor has a configuration that can use a galvanic current which is produced by metal being brought into contact with a body fluid.

As stated above, the biosensor 1 is used being swallowed. The living body 200 swallows the biosensor 1 for the analysis of the body fluids. The sites where body fluids are analyzed may be, but are not limited to, the mouth, stomach, intestines, and other organs.

The biosensor 1 performs periodic analysis at a predetermined interval or continuous analysis. The analysis results are temporarily stored in the biosensor 1, for example, and transmitted to the external device 300 through wireless communication. Thus, the patient and the physician can ascertain the measurement results.

### <Second Embodiment>

Fig. 4 is a schematic perspective view illustrating a biosensor according to a second embodiment of the present invention.

This biosensor 1 is brought into contact with a living body to obtain information relating to the living body.

When in use, the biosensor 1 is brought into contact with, for example, the skin or the tongue. When the biosensor 1 is used being brought into contact with, for example, the skin, the biosensor 1 may be pressed against the skin or may be attached to the skin.

Alternatively, the biosensor 1 may be used being embedded in the body. In this case, the biosensor 1 can be used such as for measurement of electroencephalogram, electrocardiogram or electromyogram, or measurement or acquisition of pH of gastric juice or blood.

The biosensor 1 is substantially similar to the biosensor 1 described referring to Figs. 1 and 2, excepting that the former adopts the following configuration. Namely, in this biosensor 1, the support 3 supports both electrodes 2a and 2b on one surface, with the electrodes 2a and 2b projecting from the support surface of the support 3. Use of this structure facilitates contact of the electrodes 2a and 2b with the support.

### <Third Embodiment>

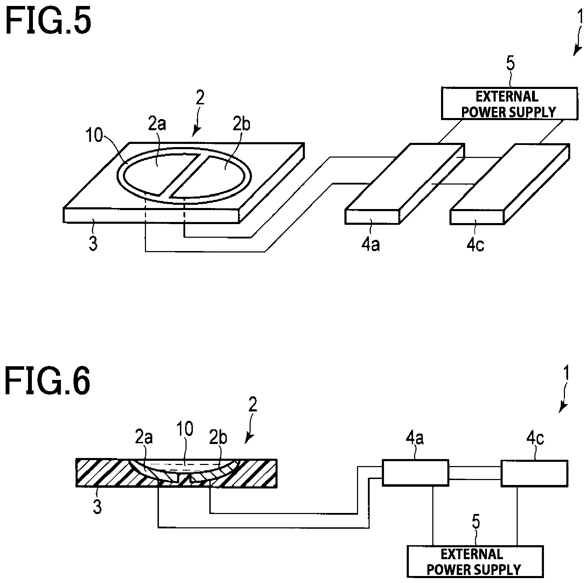

Fig. 5 is a schematic perspective view illustrating a biosensor according to a third embodiment of the present invention. Fig. 6 is diagram illustrating a cross-section of part of the biosensor of Fig. 5.

This biosensor 1 is a device that uses a body fluid collected from a living body as a sample 10, i.e., a device that is used in vitro (ex vivo).

The biosensor 1 includes an electrode unit 2 that includes a pair of electrodes 2a and 2b and a support 3. The support 3 has a plate-like shape, with an upper surface thereof being provided with a recess for holding the sample 10. The electrodes 2a and 2b are arranged in the recess.

In the biosensor 1, a signal processing unit 4a is electrically connected to the electrodes 2a and 2b and an external power supply 5, and an output unit 4c is electrically connected to the signal processing unit 4a and the external power supply 5. The signal processing unit 4a not only plays the role of the signal processing unit 4a described referring to Fig. 2, but also serves as a control unit 4b. The output unit 4c plays the role of the output unit 4c described referring to Fig. 2.

Being used in vitro, the biosensor 1 does not have to be mounted with a battery and the like, if the signal processing unit 4a and the output unit 4c are configured to be connected to the external power supply 5. The external power supply 5 may be a DC power supply or an AC power supply.

The biosensor 1 is manufactured by, for example, sequentially performing the following first to eighth processes. Since the electrodes 2a and 2b can be manufactured by the same method, description of the manufacturing method for the electrode 2b is omitted herein.

### (First process)

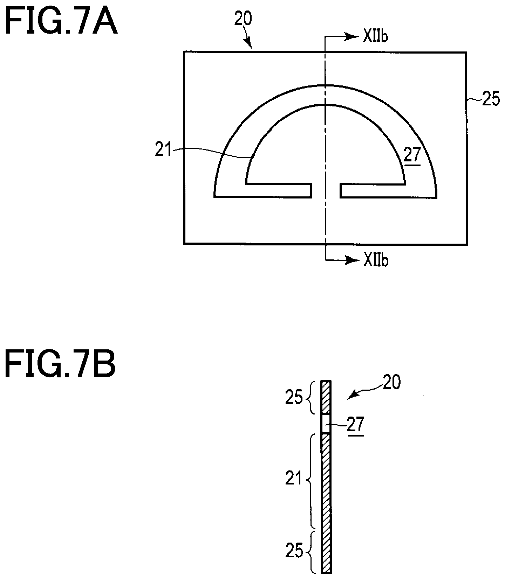

Figs. 7A and 7B are diagrams illustrating a first process in the manufacture of the biosensor shown in Figs. 5 and 6. Fig. 7A is a plan view, and Fig. 7B is a cross-sectional view taken along the line VIIb-VIIb of Fig. 7A.

In the first process, a metal plate 20 shown in Figs. 7A and 7B is used. The metal plate 20 includes a substrate 21 of the electrode 2a and a support part 25. The metal plate 20 is provided with a slit 27. The substrate 21 and the support part 25 are separated from each other via the slit 27, except for the connection portion connecting therebetween.

The material for the metal plate 20 is selected considering ease of molding, strength after molding, thermal expansion characteristics, melting point, ease of formation of oxide film, price, electrochemical formation of a carbon protective layer on the surface, and the like. Since a carbon protective layer is formed on the substrate 21, electrochemical stability or the like of the substrate 21 is not important. Examples of the material that can be used for the metal plate 20 include nickel, copper, iron, an alloy thereof, and stainless steel.

The substrate 21 herein has a plate shape but may have a different shape such as a rod shape. Further, the metal plate 20 may be subjected to press work, such as bending or drawing, to deform the substrate 21. The slit 27 may be formed by, for example, shear processing, chemical etching, blast processing, laser processing, or a combination thereof. A plurality of processes may be combined in consideration of productivity and accuracy. For example, the material may be chemically etched and then bent with a press, followed by cutting a part with a laser.

### (Second process)

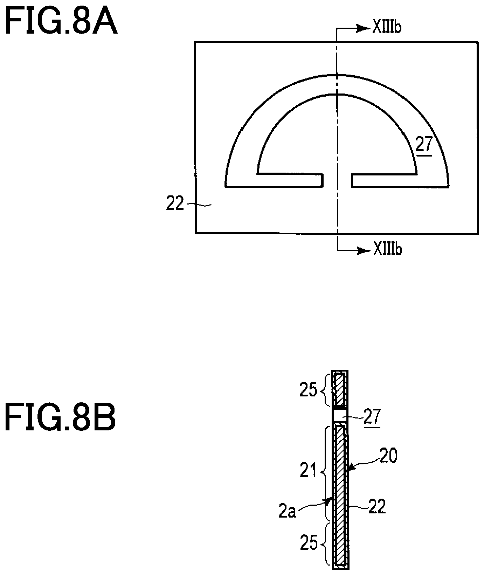

Figs. 8A and 8B are diagrams illustrating a second process in the manufacture of the biosensor shown in Figs. 5 and 6. Fig. 8A is a plan view, and Fig. 8B is a cross-sectional view taken along the line VIIIb-VIIIb of Fig. 8A.

In the second process, as shown in Figs. 8A and 8B, a carbon protective layer 22 is formed on the entire surface of the metal plate 20. For example, the protective layer 22 is formed by plating. Thus, the electrode 2a is obtained.

### (Third process)

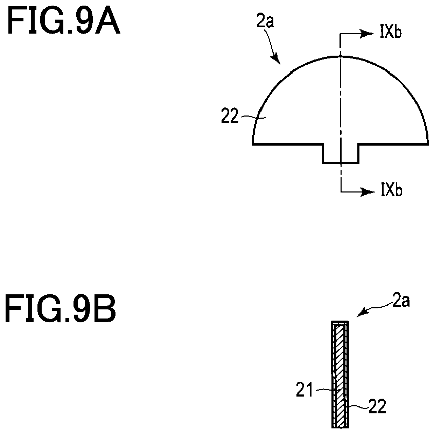

Figs. 9A and 9B are diagrams illustrating a third process in the manufacture of the biosensor shown in Figs. 5 and 6. Fig. 9A is a plan view, and Fig. 9B is a cross-sectional view taken along the line IXb-IXb of Fig. 9A.

In the third process, as shown in Figs. 9A and 9B, the substrate 21 is cut off from the support part 25 together with the protective layer 22 thereon. For example, a laser, a microcutter or the like is used to cut the joint between the substrate 21 and the support part 25.

In this way, the electrode 2a is obtained. The electrode 2b is obtained using a method similar to this. The substrate 21 of each of the electrodes 2a and 2b has an exposed surface that is the cut surface, but the rest of the surface is covered with the protective layer 22.

### (Fourth process)

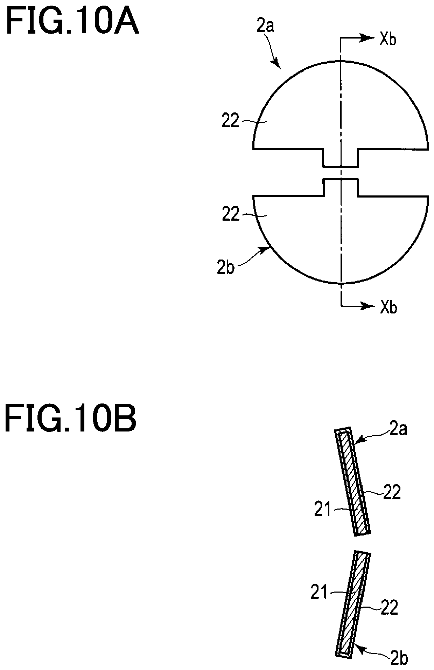

Figs. 10A and 10B are diagrams illustrating a fourth process in the manufacture of the biosensor shown in Figs. 5 and 6. Fig. 10A is a plan view, and Fig. 10B is a cross-sectional view taken along the line Xb-Xb of Fig. 10A.

In the fourth process, as shown in Figs. 10A and 10B, the electrodes 2a and 2b are temporarily secured. Herein, the electrodes 2a and 2b are arranged so that the main surfaces thereof are inclined against each other, and that the cut surfaces thereof diagonally face each other. Although not shown, the electrodes 2a and 2b are preferably temporarily secured to fixing jigs.

### (Fifth process)

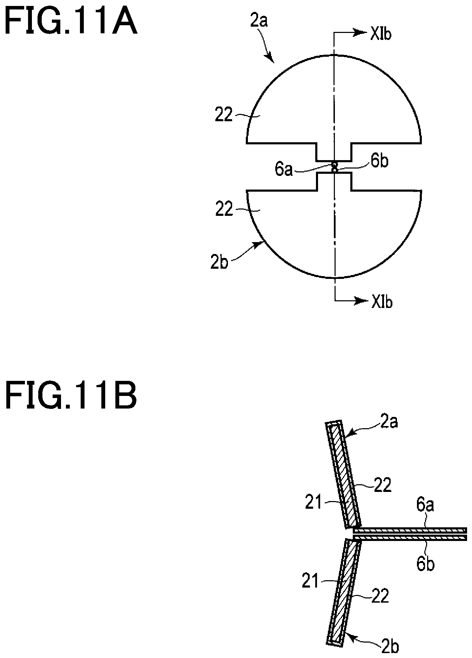

Figs. 11A and 11B are diagrams illustrating a fifth process in the manufacture of the biosensor shown in Figs. 5 and 6. Fig. 11A is a plan view, and Fig. 11B is a cross-sectional view taken along the line XIb-XIb of Fig. 11A.

In the fifth process, as shown in Figs. 11A and 11B, an end of a lead wire 6a is bonded to the electrode 2a, and an end of a lead wire 6b is bonded to the electrode 2b. The lead wires 6a and 6b are preferably bonded to the substrates 21 exposed in the respective cut surfaces. The lead wires 6a and 6b are separated from each other so as not to be electrically shorted.

### (Sixth process)

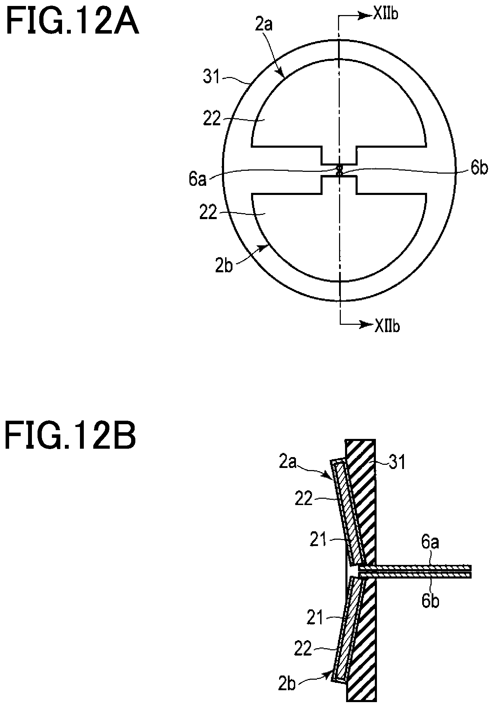

Figs. 12A and 12B are diagrams illustrating a sixth process in the manufacture of the biosensor shown in Figs. 5 and 6. Fig. 12A is a plan view, and Fig. 12B is a cross-sectional view taken along the line XIIb-XIIb of Fig. 12A.

In the sixth process, as shown in Figs. 12A and 12B, the electrodes 2a and 2b are supported by a support body 31. The support body 31 supports each of the electrodes 2a and 2b so that one of the main surfaces is exposed, and the entirety of the other main surface is covered.

The support body 31 needs to be stable toward the liquid sample used for biomedical sensing. As the material for the support body 31, an organic resin is suitable.

The liquid sample should be ensured not to permeate between the support body 31 and each of the electrodes 2a and 2b so that the effective area of the electrodes 2a and 2b remains constant. Further, from the viewpoint of preventing corrosion of the substrate 21, the liquid sample should be ensured not to contact the connection portion between the electrode 2a and the lead wire 6a and the connection portion between the electrode 2b and the lead wire 6b. Therefore, the support body 31 is preferably formed embedding these connecting portions, or an insulating layer is preferably formed on each connection portion.

### (Seventh process)

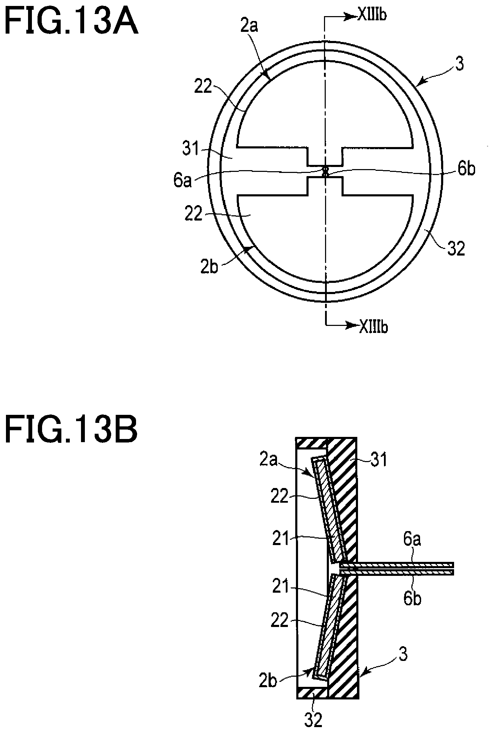

Figs. 13A and 13B are diagrams illustrating a seventh process in the manufacture of the biosensor shown in Figs. 5 and 6. Fig. 13A is a plan view, and Fig. 13B is a cross-sectional view taken along the line XIIIb-XIIIb of Fig. 13A.

In the seventh process, as shown in Figs. 13A and 13B, a wall 32 is provided to the upper surface of the support body 31 to surround the electrodes 2a and 2b. Thus, the support 3 including the support body 31 and the wall 32 is obtained.

Due to provision of the wall 32, the liquid sample is less likely to flow out. Further, provision of the wall 32 contributes to reliably bringing the liquid sample into contact with the overall upper surfaces of the electrodes 2a and 2b.

The material of the wall 32 is preferably the same as the material of the support body 31. The wall 32 may be formed in the process of forming the support body 31.

### (Eighth process)

In an eighth process, the lead wires 6a and 6b are connected to the electronic component including the signal processing unit 4a and the output unit 4c shown in Figs. 5 and 6 to electrically connect the electrodes 2a and 2b to the signal processing unit 4a. For example, when this electronic component includes one or more semiconductor chips and a printed wiring board mounting the chips thereon, the lead wires 6a and 6b are connected to terminals on the printed wiring board.

In this way, the biosensor 1 shown in Figs. 5 and 6 is obtained.

In the biosensor 1, the electrode unit 2 may be made detachable. In this case, the lead wires 6a and 6b are, for example, electrically connected to the electronic component via connectors. If the electrode unit 2 is made detachable, the electrode unit 2 alone can be changed.

### <Fourth Embodiment>

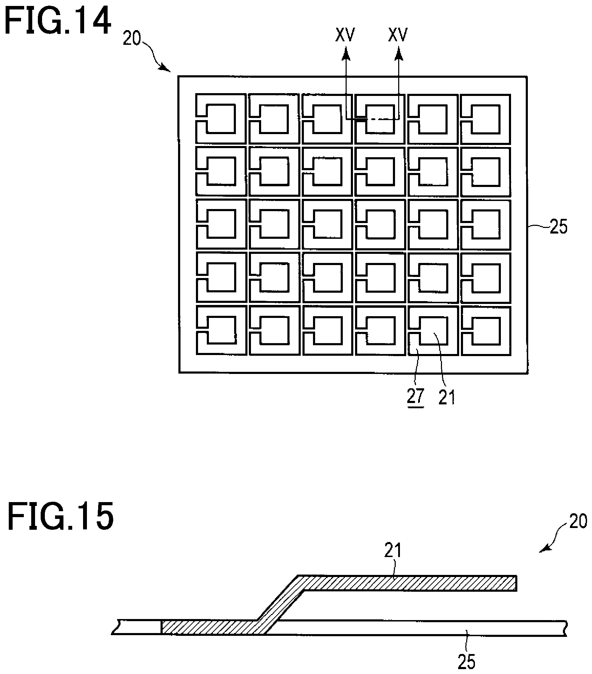

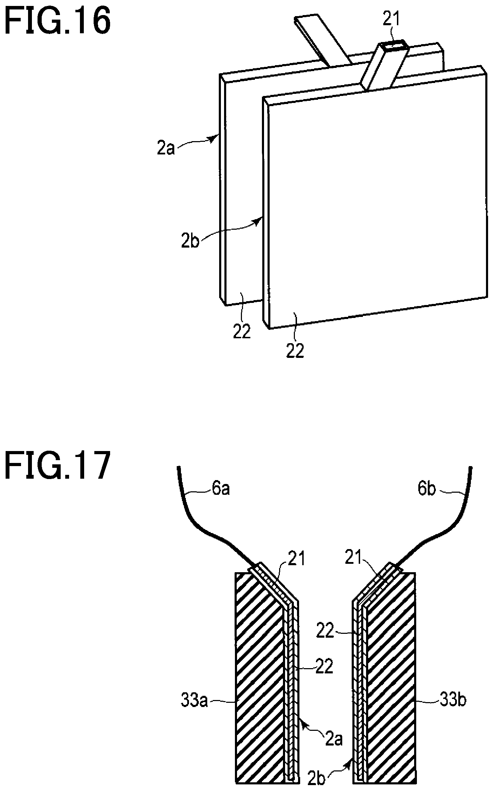

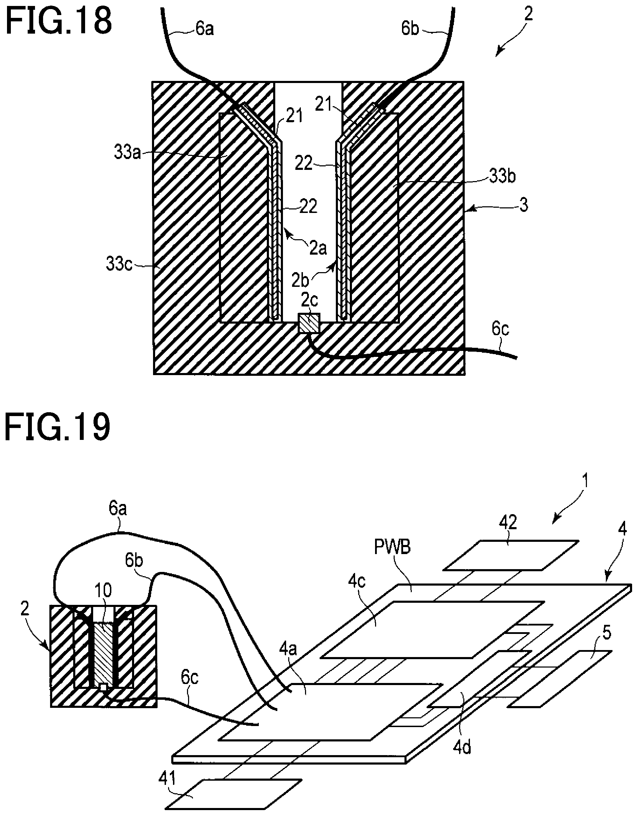

Fig. 14 is a plan view illustrating a first process in the manufacture of a biosensor according to a fourth embodiment of the present invention. Fig. 15 is a cross-sectional view taken along the line XV-XV in the structure shown in Fig. 14. Fig. 16 is a schematic perspective view illustrating an electrode obtained through a second process in the manufacture of the biosensor according to the fourth embodiment. Fig. 17 is a cross-sectional view illustrating a third process in the manufacture of the biosensor according to the fourth embodiment. Fig. 18 is a cross-sectional view illustrating a fourth process in the manufacture of the biosensor according to the fourth embodiment. Fig. 19 is a schematic diagram illustrating the biosensor according to the fourth embodiment, with part being a cross-sectional view, and the rest being a perspective view.

The biosensor 1 shown in Fig. 19 is a device that uses a body fluid collected from a living body as a sample 10, i.e., a device used in vitro (ex vivo).

As shown in Fig. 18, the biosensor 1 includes an electrode unit 2 which includes electrodes 2a, 2b and 2c, a support 3 and lead wires 6a, 6b and 6c.

The electrodes 2a and 2b are measurement electrodes. The electrodes 2a and 2b each include a substrate 21 and the protective layer 22.

The electrode 2c is a reference electrode. For example, a silver / silver chloride electrode can be used as the reference electrode. When obtaining a reference electrode, a component having a metallic silver surface is used. Specifically, the metallic silver surface is chlorinated, and silver chloride is produced thereon.

The support 3 has a recess. The support 3 supports the electrodes 2a and 2b on the sidewalls of the recess so that the electrodes 2a and 2b face each other. Also, the support 3 supports the electrode 2c on the bottom of the recess.

An end of the lead wire 6a is bonded to the substrate 21 of the electrode 2a. An end of the lead wire 6b is bonded to the substrate 21 of the electrode 2b. An end of the lead wire 6c is bonded to the electrode 2c. Other ends of the lead wires 6a, 6b and 6c are electrically connected to a signal processing unit 4a shown in Fig. 19.

As shown in Fig. 19, the biosensor 1 includes an electronic component 4 which includes the signal processing unit 4a, a power supply unit 4d and an output unit 4c. The signal processing unit 4a is electrically connected to an interface 41 to receive input of data. The signal processing unit 4a, not only plays the role of the signal processing unit 4a described referring to Fig. 2, but also plays a role of a control unit 4b. The power supply unit 4d is electrically connected to an external power supply 5, the signal processing unit 4a and the output unit 4c. The output unit 4c is electrically connected to the signal processing unit 4a, and an interface 42 from which data is externally transmitted.

For example, the biosensor 1 is manufactured by the following method.

First, a metal plate 20 shown in Figs. 14 and 15 is prepared. The metal plate 20 is obtained by providing a slit 27 in a flat metal plate to form a substrate 21 and a support part 25, followed by bending to uplift the substrate 21 with respect to the support part 25.

Then, a protective layer 22 is formed and the support part 25 is cut off from each of the electrodes 2a and 2b through a method similar to one described in the third embodiment. As shown in Fig. 16, the electrodes 2a and 2b obtained in this manner each have a cut surface where the substrate 21 is exposed.

Then, the electrodes 2a and 2b are respectively supported by insulating components 33a and 33b. For example, the electrode 2a is supported by the insulating component 33a, with a main surface thereof being exposed, and the entirety of the other main surface thereof being covered with the insulating component 33a. Further, for example, the electrode 2b is supported by the insulating component 33b, with a main surface thereof being exposed, and the entirety of the other main surface being covered with the insulating component 33b.

Then, an end of the lead wire 6a and an end of the lead wire 6b shown in Fig. 17 are respectively bonded to the electrodes 2a and 2b through a method similar to one described in the third embodiment. The lead wires 6a and 6b are each bonded to the exposed part of the substrate 21.

Then, the electrode 2a integrated with the insulating component 33a and the electrode 2b integrated with the insulating component 33b are placed in the cavity of a mold, not shown, such that the exposed main surfaces face each other, being interposed by the core of the mold, not shown. In this case, the electrode 2c shown in Fig. 18 is placed in advance beneath the core.

Then, a resin is injected into the mold. The resin is cured to obtain a support body 33c shown in Fig. 18. Then, the mold is disassembled to obtain the electrode unit 2 shown in Fig. 18.

Then, as shown in Fig. 19, the electrode unit 2 is electrically connected to the electronic component 4 to obtain the biosensor 1.

In the third and fourth embodiments, the lead wire 6a or 6b is bonded to an exposed metal surface of the substrate 21. A different method may be used for electrically connecting between the electrode 2a and the lead wire 6a and between the electrode 2b and the lead wire 6b.

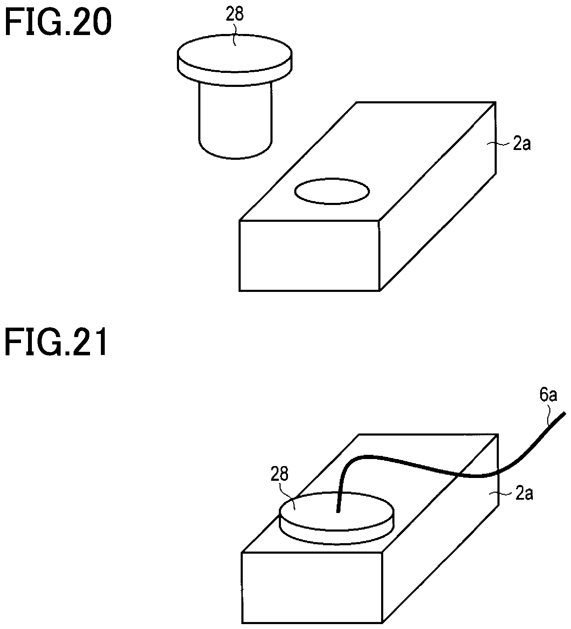

Fig. 20 is a schematic perspective view illustrating a first process of a connection method according to a modification. Fig. 21 is a schematic perspective view illustrating a second process of the connection method according to the modification.

In this method, first, a through hole is provided to a substrate having a metal surface. Then, a protective layer is formed on the entirety of the exposed part of the metal surface. Thus, an electrode 2a as shown in Fig. 20 is obtained. When the sidewall of the through hole provided to the substrate is made of a metal, the protective layer 22 is preferably formed covering not only the outer surface of the substrate, but also the sidewall of the through hole.

Then, as shown in Fig. 21, a pin 28 shown in Fig. 20 is inserted into the through hole. A different component, such as a screw, may be used in place of the pin 28.

Then, the lead wire 6a is bonded to the pin 28. The lead wire 6a may be bonded to the pin 28 prior to the pin 28 being inserted in the through hole. The aforementioned protective layer may also be formed on the surface of the pin 28.

In this way, electrical connection is established between the electrode 2a and the lead wire 6a. Similarly, electrical connection is established between the electrode 2b and the lead wire 6b.

The electrodes 2a and 2b may be electrically connected to other conductive members through the following method.

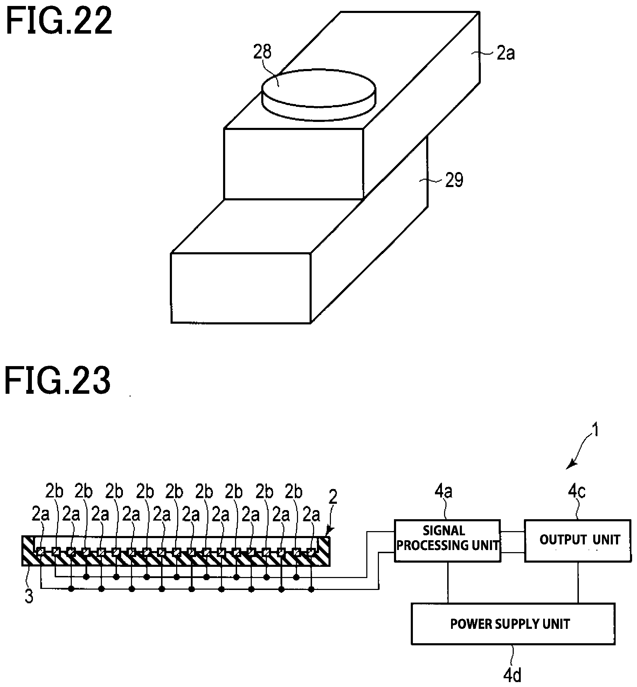

Fig. 22 is a schematic perspective view illustrating a connection method according to another modification.

In this method, first, a through hole is provided to a substrate having a metal surface. Then, a protective layer is formed on the entirety of the exposed part of the metal surface. Thus, an electrode 2a provided with a through hole is obtained. When the sidewall of the through hole provided to the substrate is made of a metal, the protective layer is preferably formed covering not only the outer surface of the substrate, but also the sidewall of the through hole.

Then, as shown in Fig. 22, the electrode 2a is overlaid on the separately prepared substrate 29. According to an example, an aperture communicating with the through hole of the electrode 2a is provided in advance to the substrate 29. For example, the substrate 29 may be a conductive substrate having a metal surface. Such a substrate 29 may be entirely made of metal or may be an insulator covered with a metal layer. The aforementioned protective layer may also be formed on the surface of the substrate 29.

Then, a pin 28 is inserted into the through hole of the electrode 2a and the aperture of the substrate 29 in the state where the electrode 2a is overlaid on the substrate 29. Thus, the electrode 2a is integrated with the substrate 29, with an electrical connection being established therebetween.

In place of the pin 28, a different component, such as a screw, may be used for the integration. The electrode 2b can be manufactured and integrated with the substrate 29 through a method similar to one used for the electrode 2a.

According to the aforementioned method, the electrodes 2a and 2b can be made detachable from the sensor body. Therefore, for example, at least one of the electrodes 2a and 2b can be made disposable.

Further, the protective layer can be uniformly formed on the sidewall of the through hole by plating. Namely, this method has an advantage of not exposing the metal surface.

### <Fifth Embodiment>

Fig. 23 is a schematic diagram illustrating a biosensor according to a fifth embodiment of the present invention.

This biosensor 1 is a device that uses a body fluid collected from a living body as a sample 10 similarly to the biosensors of the third and fourth embodiments, i.e., a device used in vitro (ex vivo). The biosensor 1 is different from the biosensors of the third and fourth embodiments in that it includes a plurality of sets of the electrode 2a and the electrode 2b.

In Fig. 23, the electrodes 2a are electrically connected to each other, and the electrodes 2b are electrically connected to each other; however, alternatively, the electrodes 2a may be electrically insulated from each other, and the electrodes 2b may be electrically insulated from each other. In the latter case, if the surfaces of some electrode pairs are differently modified from those of other electrode pairs, different biomarkers can be detected from a single liquid sample. The surface modification may be given to both the electrodes 2a and 2b, or may be given to only one of them.

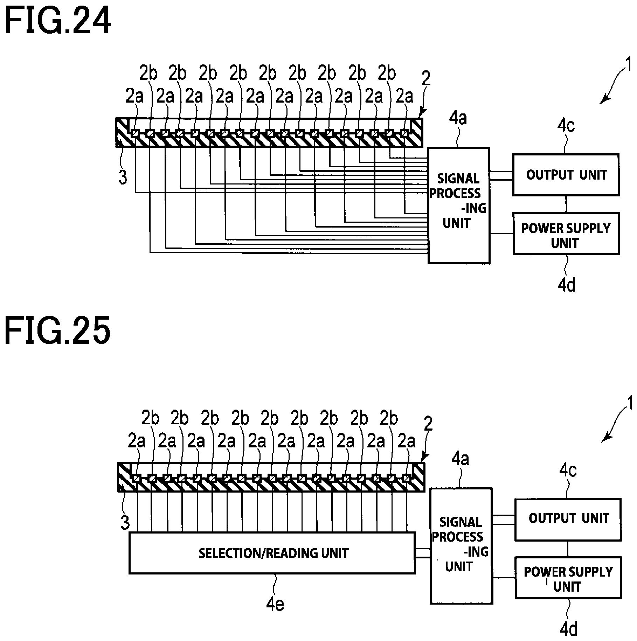

Fig. 24 is a schematic diagram illustrating a modification of the biosensor shown in Fig. 23. In the biosensor 1 shown in Fig. 23, the electrodes 2a are electrically connected to each other, and the electrodes 2b are electrically connected to each other. In this regard, in the biosensor 1 shown in Fig. 24, the electrodes 2a are electrically insulated from each other, and are separately electrically connected to the signal processing unit 4a. Also, the electrodes 2b are electrically insulated from each other, and are separately electrically connected to the signal processing unit 4a. With this configuration, for example, a plurality of electrode sets can be provided, with each set being an electrode 2a paired with any one of the electrodes 2b, to obtain different signals.

Fig. 25 is a schematic diagram illustrating another modification of the biosensor shown in Fig. 23. The biosensor 1 shown in Fig. 25 is similar to the biosensor 1 shown in Fig. 23, excepting that the former further includes a selection/reading unit 4e.

The selection/reading unit 4e is electrically connected to the electrodes 2a and 2b and to the signal processing unit 4a. For example, the selection/reading unit 4e selects a set of an electrode 2a and an electrode 2b, and supplies the signal obtained from these electrodes 2a and 2b to the signal processing unit 4a. The selection/reading unit 4e may sequentially select a set of an electrode 2a and an electrode 2b, and consecutively supply the signals obtained from the electrodes 2a and 2b to the signal processing unit 4a.

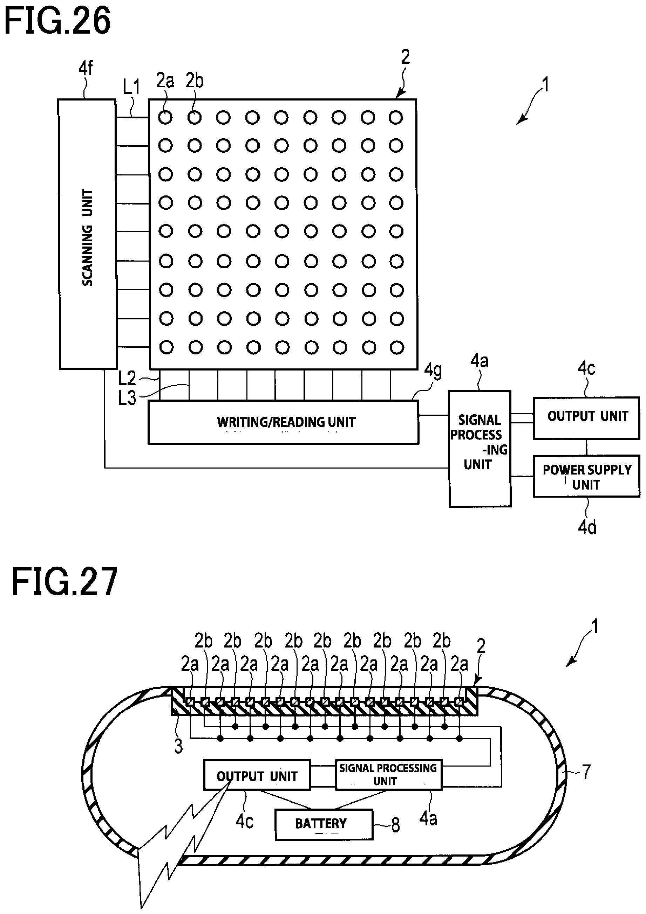

Fig. 26 is yet another modification of the biosensor shown in Fig. 23. The biosensor 1 shown in Fig. 26 is similar to the biosensor 1 shown in Fig. 23, except for the following points.

This biosensor 1 includes a plurality of scanning lines L1, a plurality of signal lines L2, a plurality of power supply lines L3, and a plurality of selection circuits (not shown).

The plurality of scanning lines L1 extend in the X direction and are arrayed in the Y direction intersecting the X direction. The plurality of signal lines L2 extend in the Y direction and are arrayed in the X direction. These signal lines L2 are electrically insulated from the scanning lines L1, while being arranged intersecting the scanning lines L1.

Intersections of the scanning lines and the signal lines are provided with respective pixel circuits to which a plurality of power supply lines or a common power supply line are connected. Electrodes 2a, 2b are connected to each pixel circuit.

Each pixel circuit has a selection transistor in which the gate electrode is connected to the scanning line, one of source and drain electrodes is connected to the signal line, and the other of the source and drain electrodes is connected to the pixel circuit. When an on signal is supplied to the scanning lines L1, each selection transistor connects the pixel circuit to the signal line, and outputs the signal in the pixel circuit to the writing/reading unit. While an off signal is supplied to the scanning lines L1, each pixel circuit is insulated from the signal line L2, that is, each pixel circuit is disconnected from the writing/reading unit.

This biosensor 1 further includes a scanning unit 4f and a writing/reading unit 4g.

The scanning unit 4f is electrically connected to the signal processing unit 4a and the scanning lines L1. The scanning unit 4f sequentially selects the scanning lines L1, supplies an on signal to the selected scanning line L1, and supplies an off signal to the unselected scanning lines L1, based on a control signal supplied from the signal processing unit 4a.

The writing/reading unit 4g is connected to the signal processing unit 4a and the signal lines L2. For example, as a writing operation, the writing/reading unit 4g writes a voltage setting or a current setting, as electrode bias conditions, for the pixel circuit whose scanning line has been selected. As a reading operation, the writing/reading unit 4g reads a current signal and a voltage signal from the pixel circuit whose scanning line has been selected, according to the bias conditions.

For example, this biosensor 1 line-sequentially drives the electrode unit to read signals. In this case, for example, the biosensor 1 can obtain signals the number of which is equal to the product of the number M of the scanning lines L1 and the number N of the signal lines L2. Namely, the biosensor 1 can obtain signals from two-dimensionally distributed M×N measurement points. Therefore, for example, the two-dimensional distribution of the signal intensity can be obtained. Further, the biosensor 1 can repeatedly read signals to examine temporal change of the two-dimensional distribution.

Thus, the state of fluidity and diffusion of the liquid sample can be detected. The biosensor 1 may be used for detecting the components involved in the metabolism in cells, i.e., the ground substances which are metabolic starting materials, or the metabolites which are the final products or intermediate products of metabolism. In this case, the total amount of the above components can be estimated from the signals obtained from the individual pairs of electrodes each made up of an electrode 2a and an electrode 2b.

This biosensor 1 can detect different biomarkers from a single liquid sample if the surfaces of some electrode pairs are differently modified from those of other electrode pairs.

In this biosensor 1, part of the electrode pairs can be used for measurement, and another part of the electrode pairs can be used for calibration. For example, only part of the electrode pairs may be modified in the surfaces, so that the signals obtained from the surface-modified electrode pairs can be calibrated with the signals obtained from the electrode pairs that are not surface-modified. For example, this way of calibration can reduce the influence given to the measurement results by the environmental change, such as temperature change, or the manufacture variation.

In this biosensor 1, there is a probability that energization may cause characteristic change in at least one of the electrodes 2a and 2b. For example, energization may cause at least one of the electrodes 2a and 2b to deteriorate or irreversibly adsorb substances contained in the liquid sample. To cope with this, the electrode pairs used for measurement may be sequentially changed to prolong the life of the biosensor 1. For example, part of the electrode pairs may be used for measurement first, and then other part of the electrode pairs may be used for measurement.

The electrode pairs are disposed so that the measurement points are planarly distributed. Alternatively, the electrode pairs may be disposed so that the measurement points are spatially distributed. In this case, a two-dimensional distribution of the signal intensity can be obtained.

### <Sixth Embodiment>

Fig. 27 is a schematic diagram illustrating a biosensor according to a sixth embodiment of the present invention.

This biosensor 1 is used being swallowed in the same manner as the biosensor according to the first embodiment.

The biosensor 1 includes a housing 7 which supports an electrode unit 2 such that electrodes 2a and 2b are exposed. The housing 7 incorporates a signal processing unit 4a, an output unit 4c, and a battery 8.

The biosensor 1 includes a plurality of sets of an electrode 2a and an electrode 2b similarly to the biosensor of the fifth embodiment. In Fig. 27, the electrodes 2a are electrically connected to each other, and the electrodes 2b are electrically connected to each other. Alternatively, the electrodes 2a may be electrically insulated from each other, and the electrodes 2b may be electrically insulated from each other.

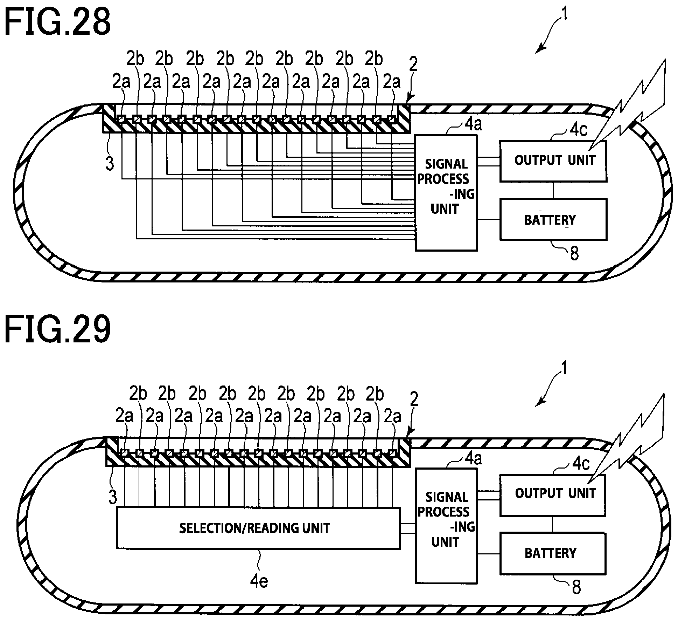

Fig. 28 is a schematic diagram illustrating a modification of the biosensor shown in Fig. 27. The biosensor 1 shown in Fig. 27 includes a plurality of sets of electrodes 2a and 2b similarly to the biosensor of the fifth embodiment. Moreover, in Fig. 27, the electrodes 2a are electrically connected to each other, and the electrodes 2b are electrically connected to each other. In contrast, in the biosensor 1 shown in Fig. 28, the electrodes 2a are electrically insulated from each other, while being separately electrically connected to the signal processing unit 4a, and the electrodes 2b are electrically insulated from each other, while being separately electrically connected to the signal processing unit 4a. With this configuration, for example, a plurality of electrode sets can be provided, with each set being an electrode 2a paired with any one of the electrodes 2b, to obtain different signals.

Fig. 29 is a schematic diagram illustrating another modification of the biosensor shown in Fig. 27. The biosensor 1 shown in Fig. 29 is similar to the biosensor 1 shown in Fig. 27, excepting that the former further includes a selection/reading unit 4e.

The selection/reading unit 4e is electrically connected to the electrodes 2a and 2b and to the signal processing unit 4a. For example, the selection/reading unit 4e selects a set of an electrode 2a and an electrode 2b, and supplies a signal obtained from the electrodes 2a and 2b to the signal processing unit 4a. The selection/reading unit 4e may sequentially select a set of an electrode 2a and an electrode 2b, and consecutively supply the signals obtained from the electrodes 2a and 2b to the signal processing unit 4a.

The electrode units 2 shown in Figs. 23 and 27 are manufactured by, for example, sequentially performing the following first to fourth processes.

### (First process)

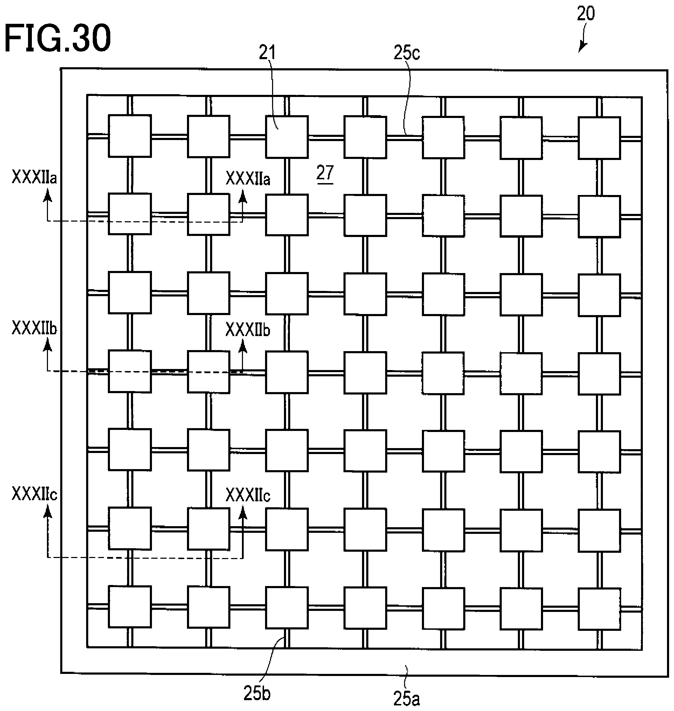

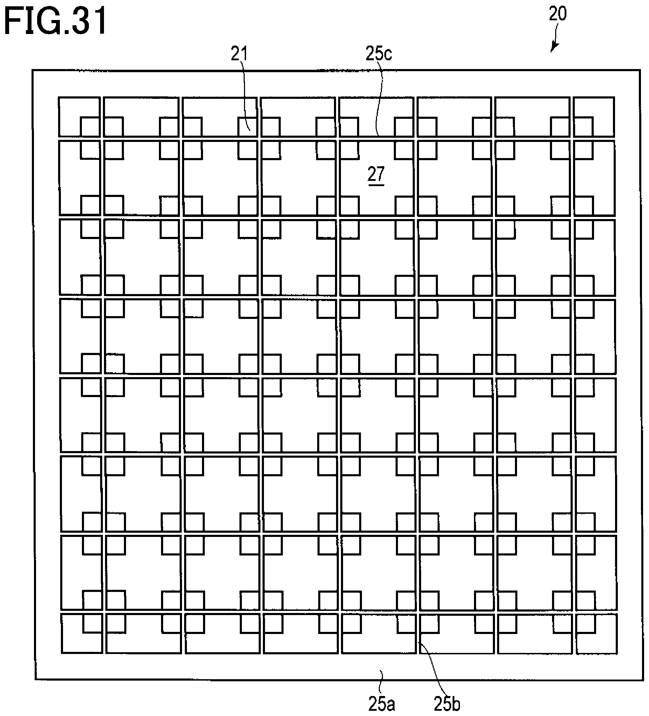

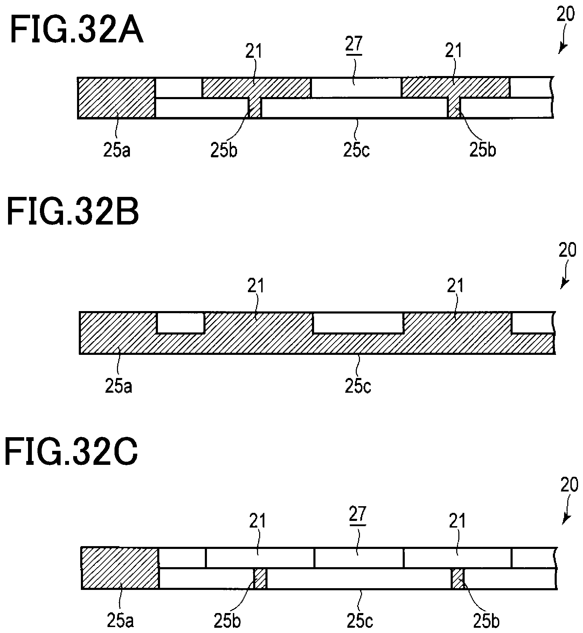

Fig. 30 is a schematic plan view illustrating a first process of a method available for the manufacture of the electrodes included in the biosensors shown in Figs. 23 and 27. Fig. 31 is a plan view of the structure shown in Fig. 30 as viewed from the back. Fig. 32A is a cross-sectional view taken along the line XXXIIa-XXXIIa in the configuration shown in Fig. 30. Fig. 32B is a cross-sectional view taken along the line XXXIIb-XXXIIb in the configuration shown in Fig. 30. Fig. 32C is a cross-sectional view taken along the line XXXIIc-XXXIIc in the configuration shown in Fig. 30.

When manufacturing the electrode unit 2 shown in Figs. 23 and 27, first, the metal plate 20 shown in Figs. 30, 31, and 32A to 32C is prepared.

The metal plate 20 includes longitudinally and transversely arrayed substrates 21, and a support part supporting these substrates 21. The metal plate 20 has a slit 27.

The support part includes a frame part 25a, and linear parts 25b and 25c.

The linear parts 25b extend in the longitudinal direction and are arrayed in the transverse direction. The linear parts 25c extend in the transverse direction and are arrayed in the longitudinal direction. The linear parts 25b and 25c form a lattice. The thicknesses of the linear parts 25b and 25c are equal to each other but are smaller than the thickness of the frame part 25a.

The substrates 21 are located on the respective lattice points of the lattice formed by the linear parts 25b and 25c. The sum of the thickness of each substrate 21 and the thickness of each linear part 25b or 25c is equivalent to the thickness of the frame part 25a.

In the first process, the metal plate 20 is manufactured by, for example, the following method.

First, a first recess is formed on a surface of a flat metal plate. The first recess is formed at positions of the linear parts 25b and 25c and the slit 27.

Then, a second recess is formed on the other surface of the above flat plate. The second recess is formed at positions which do not correspond to any of the frame part 25a or the linear parts 25b and 25c. The second recess is formed so as to be connected with the first recess at a position of the slit 27.

Thus, the metal plate 20 is obtained.

### (Second process)

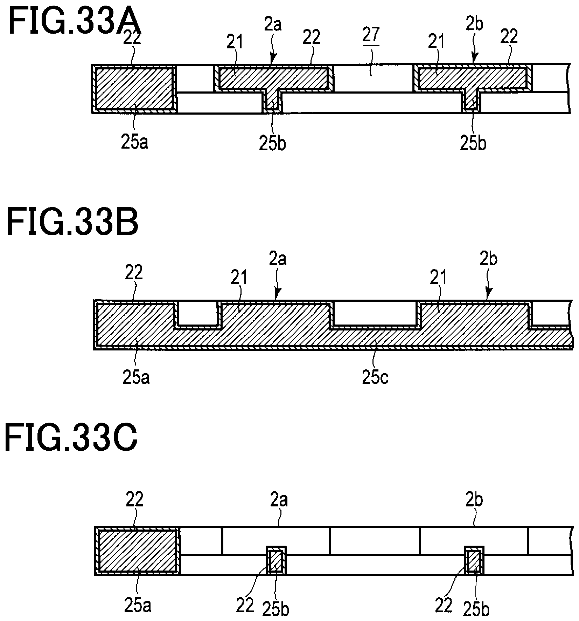

Figs. 33A to 33C are schematic diagrams illustrating a second process of the method available for the manufacture of the electrode included in the biosensors shown in Figs. 23 and 27. Figs. 33A, 33B and 33C are cross-sectional views respectively corresponding to Figs. 32A, 32B and 32C.

In the second process, a protective layer 22 is formed on the entire surface of the metal plate 20, as shown in Figs. 33A to 33C. The protective layer 22 is formed by a method similar to one described in the third embodiment. Thus, the electrodes 2a and 2b are obtained. Herein, the electrodes 2a and 2b are arrayed in a checkerboard pattern. The electrodes 2a and 2b may be arrayed in a different pattern.

### (Third process)

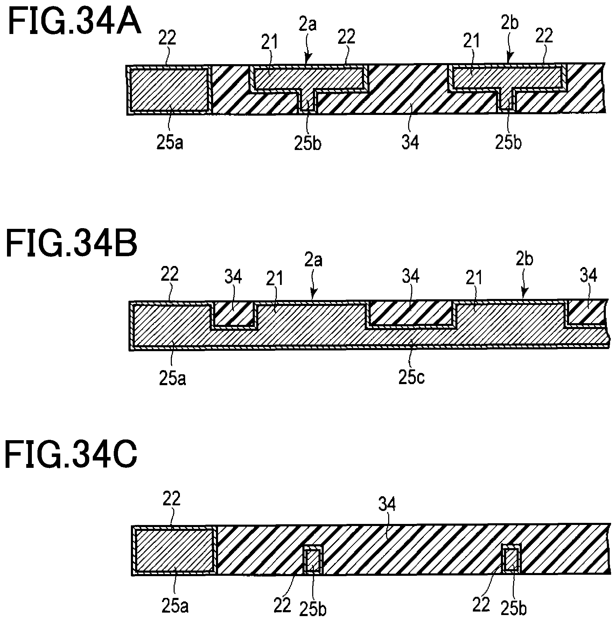

Figs. 34A to 33C are schematic diagrams illustrating a third process of the method available for the manufacture of the electrode included in the biosensors shown in Figs. 23 and 27. Figs. 34A, 34B and 34C are cross-sectional views respectively corresponding to Figs. 32A, 32B and 32C.

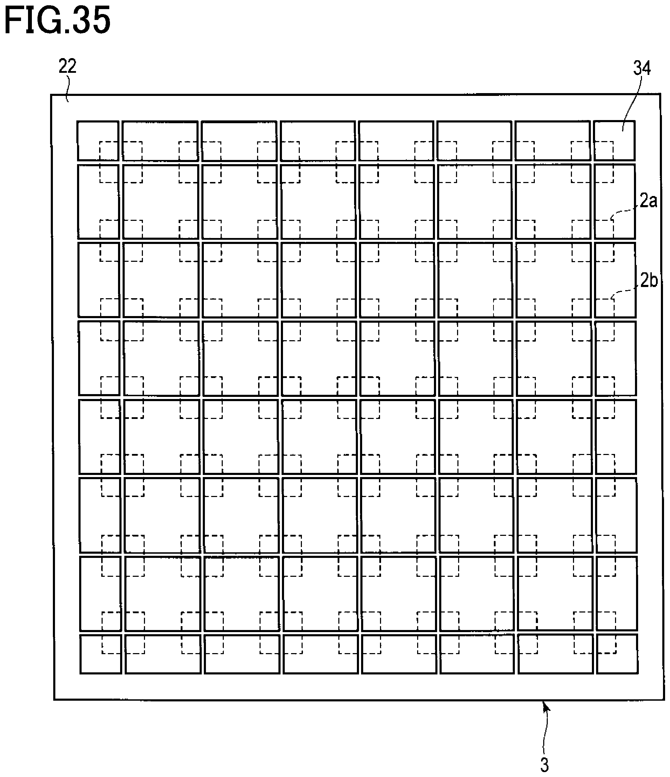

Fig. 35 is a schematic diagram illustrating the third process of the method available for the manufacture of the electrode included in the biosensors shown in Figs. 23 and 27, that is, a plan view of the structure obtained through the third process, as viewed from the back.

In the third process, as shown in Figs. 34A to 34C and Fig. 35, the first and second recesses are filled with a support layer 34 made of a resin. Thus, a support 3 made up of the support layer 34 and the frame part 25a, and supporting the electrodes 2a and 2b is formed.

### (Fourth process)

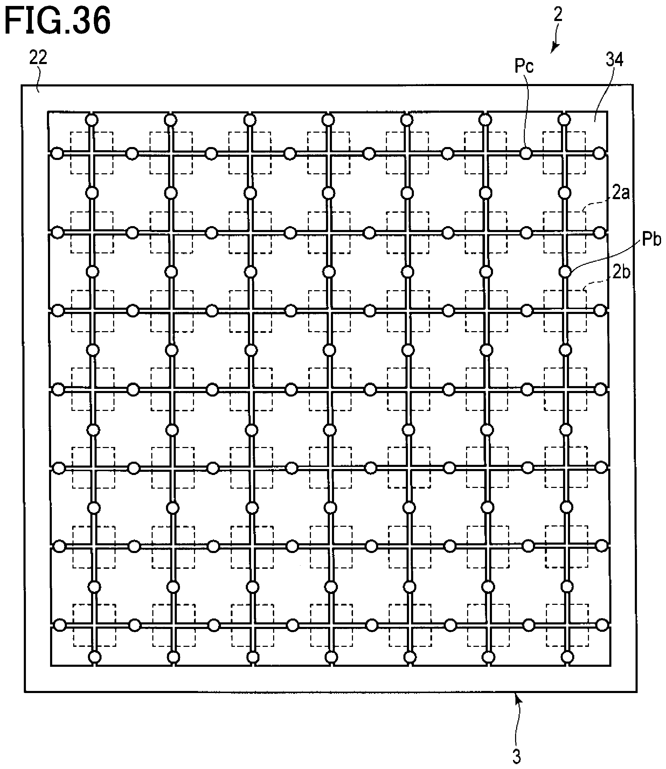

Fig. 36 is a schematic diagram illustrating a fourth process of the method available for the manufacture of the electrode included in the biosensors shown in Figs. 23 and 27, that is, a plan view of the structure obtained through the fourth process, as viewed from the back.

In the fourth process, as shown in Fig. 36, each linear part 25b is cut at positions Pb, and each linear part 25c is cut at positions Pc. Thus, the electrodes 2a and 2b are electrically insulated from each other.

Thus, the electrode unit 2 shown in Fig. 23 or 27 is obtained. The electrode unit 2 may be provided with the wall 32 described referring to Fig. 13B. The electrode unit 2 obtained in this manner can be made flexible by making the support layer 34 and the frame part 25a flexible.

### <Seventh Embodiment>

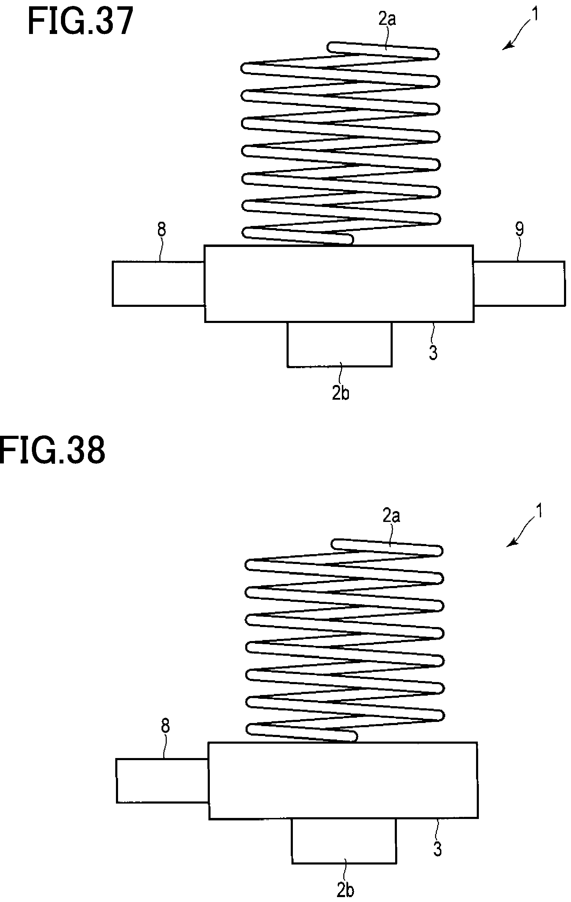

Figs. 37 to 39 are schematic side views illustrating an example of a biosensor according to a seventh embodiment of the present invention.

The biosensor 1 shown in Fig. 37 includes electrodes 2a and 2b, a support 3, a battery 8, and a storage/output unit 9.

The electrode 2a has a coil shape and can serve as a spring.

The electrode 2b has a columnar shape and is located in the expanding direction of the electrode 2a.

The support 3, which is located between the electrodes 2a and 2b, has a hollow structure and incorporates an electronic component that includes a signal processing unit.

The battery 8 is detachably mounted to the support 3. The battery 8 supplies electric power to the electronic component incorporated in the support 3.

The storage/output unit 9 is mounted to the support 3. The storage/output unit 9 incorporates a storage device to store information generated by the signal processing unit. The storage/output unit 9 transmits this information to an external device in a wireless or wired fashion.

The biosensor 1 shown in Fig. 38 includes electrodes 2a and 2b, a support 3 and a battery 8. This biosensor 1 does not include a storage/output unit 9 and is similar to the biosensor described referring to Fig. 37, except that the electronic component incorporated in the support 3 further includes an output unit and the like.

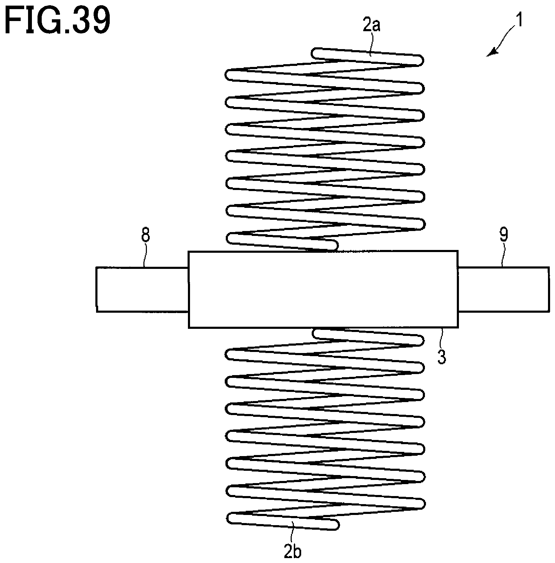

The biosensor 1 shown in Fig. 39 is similar to the biosensor described referring to Fig. 37, except that the electrode 2b has a configuration similar to the electrode 2a. In the biosensor 1 shown in Fig. 38 as well, the electrode 2b may have a structure similar to the electrode 2a. Further, at least one of the electrodes 2a and 2b may have a leaf-spring shape and may serve as a spring.

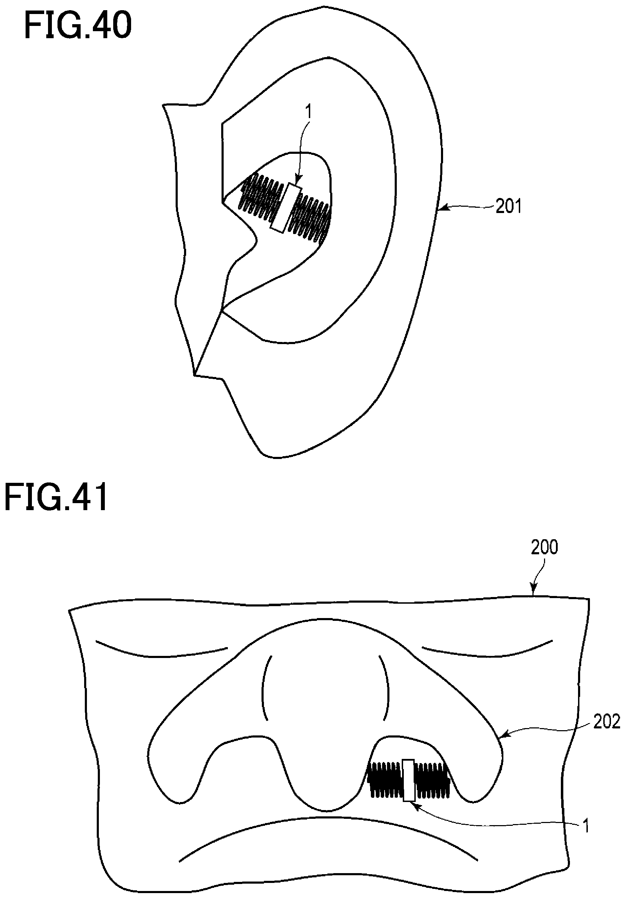

Fig. 40 is a schematic diagram illustrating a usage example of the biosensor according to the seventh embodiment of the present invention. Fig. 41 is a schematic diagram illustrating another usage example of the biosensor according to the seventh embodiment of the present invention.

As shown in Fig. 40, the biosensor 1 may be arranged in an ear 201. When the biosensor 1 is arranged in the ear 201, for example, sweat can be used as the liquid sample.

As shown in Fig. 41, the biosensor 1 may be arranged in a nose 202. When the biosensor 1 is arranged in the nose 202, for example, nasal mucous can be used as the liquid sample. In this case, at least one of the electrodes 2a and 2b may carry an enzyme to detect pathogens or viruses.

The biosensor 1 may also incorporate a temperature sensor therein. Arranging the biosensor 1 in the ear 201 or the nose 202 and constantly measuring the body temperature, menstrual cycle, pregnancy or the like can be characterized.

The biosensor 1 may incorporate a heart rate meter therein. Arranging the biosensor 1 in the ear 201 or the nose 202 and constantly measuring the heart rate, arrhythmia or the like can be examined.

The biosensor 1 may further have a function of charging itself or the surroundings thereof. For example, when the biosensor 1 is arranged in the nose 202, and the nose 202 is positively charged on the inside thereof, pollens are prevented from entering the nose 202. When the biosensor 1 is arranged in the nose 202, and the biosensor 1 or the electrode 2a or 2b is negatively charged, pollens can be captured to prevent entry deep inside the nose. When the biosensor 1 is arranged in the ear 201 or the nose 202, and the living body 200 is negatively charged, pollens can be adsorbed by the body to prevent entry into the nose 202.

The biosensor 1 may further have a function of generating electromagnetic waves. Pollens can be decomposed using electromagnetic waves.

The biosensor 1 arranged in the ear 201 may further have a function of a hearing aid or an earphone.

Manufacture of the biosensor and specific measurement examples using the biosensor will be described below.

### <Example 1>

In the present example, a biosensor was manufactured by the method described below, and measurements were conducted using the biosensor.

### (A) Processing of metal materials

A stainless-steel (SUS304) plate with a thickness of 0.3 mm was processed to obtain the metal plate 20 shown in Figs. 14 and 15. Specifically, this processing was performed by the following method. First, a photosensitive etching resist was applied to both surfaces of the stainless-steel plate. These resist layers were subjected to pattern exposure and development to form resist patterns on both surfaces of the stainless-steel plate. Then, these resist patterns were used as an etching mask to etch the stainless steel. Ferric chloride solution was used as the etching liquid. Then, the resist patterns were stripped to obtain a metal plate 20 having a slit 27. Then, the metal plate 20 was subjected to bending to uplift the substrates 21 with respect to the support part 25. Herein, the square portion of each substrate 21 had a dimension of 3.3 mm × 3.3 mm, and the substrates 21 were uplifted by 0.5 mm with respect to the support part 25.

### (B) Formation of protective layer

Then, a continuous carbon film with a thickness of 3 µm was formed as the protective layer 22 shown in Fig. 16 by plating on the entirety of the surface of the metal plate 20. Specifically, the protective layer 22 was formed by immersing the metal plate 20 in a molten salt containing calcium carbide (LiCl-KCl-CaCl₂: 500°C), followed by anode polarization.

### (C) Temporary arrangement of electrode and connection of lead wires

Then, the substrates 21 formed with the protective layer 22 were separated from the support part 25. Specifically, a laser beam was irradiated to the connection portion between each of the substrates 21 and the support part 25 to cut off the substrates 21 from the support part 25. The electrodes 2a and 2b shown in Fig. 16 were obtained in this manner.

Then, the insulating components 33a and 33b shown in Fig. 17 were adhered to jigs, not shown. Then, the electrodes 2a and 2b were respectively pressure-bonded to the insulating components 33a and 33b. Herein, the distance between the electrodes 2a and 2b was about 2 mm.

Then, an end of the lead wire 6a and an end of the lead wire 6b shown in Fig. 17 were respectively bonded to the electrode 2a and the electrode 2b. Copper wires were used as the lead wires 6a and 6b. The lead wires 6a and 6b were bonded to exposed parts of the respective substrates 21 using a conductive paste.

### (D) Resin molding

Then, the electrode 2a integrated with the insulating component 33a and the electrode 2b integrated with the insulating component 33b were placed in the cavity of a mold, not shown, such that the exposed main surfaces face each other being interposed by the core of the mold, not shown. In this case, the electrode 2c shown in Fig. 18 was placed beneath the core. Further, the jigs to which the insulating components 33a and 33b had been adhered were removed.

Then, a resin was injected into the mold. Curing the resin, the support body 33c shown in Fig. 18 was obtained. Then, the mold was disassembled to obtain the electrode unit 2 shown in Fig. 18. The capacity of the space produced by removing the core was 20 µL.

### (E) Preparation of electronic component

The printed wiring board PWB shown in Fig. 19 was used. A semiconductor module as a signal processing unit 4a and an output unit 4c, and a semiconductor module as a power supply unit 4d were mounted on the printed wiring board PWB. Thus, an electronic component 4 was obtained.

### (F) Connection of components

The signal processing unit 4a of the electronic component 4 was electrically connected with an interface 41 for inputting data to the signal processing unit 4a, and with the lead wires 6a, 6b and 6c. Further, the output unit 4c of the electronic component 4 was electrically connected with an interface 42 for externally transferring data from the output unit 4c. Furthermore, the power supply unit 4d of the electronic component 4 was electrically connected with an external power supply 5. In this way, the biosensor 1 shown in Fig. 19 was completed.

### (G) Measurement

10 µL of blood was mixed with 10 µL of physiological saline containing glucose oxidase and ferricyanide ions each at a concentration of 0.01 mol/L. 1 minute after start of mixing, this mixture was introduced into the recess of the electrode unit 2, and a voltage of 0.5 V was applied across the electrodes 2a and 2b. Then, 10 seconds after start of the application of the voltage, the current flowing across the electrodes 2a and 2b was measured. The current as a result of this measurement was 1 to 10 µA at a blood glucose concentration in the range of 10 to 500 mg/dL. Thus, a good current linearity was found to be obtained. This measurement method is comparatively common as a detection method of blood glucose concentration. Through the above examination, it was found that accurate measurements can be conducted using the aforementioned biosensor.

### <Example 2>

In the present example, a biosensor was manufactured by the method described below, and measurements were conducted using the biosensor.

### (A) Processing of metal materials

A stainless-steel (SUS304) plate with a thickness of 0.3 mm was processed to obtain the metal plate 20 shown in Figs. 30, 31 and 32A to 32C. Specifically, this processing was performed by the following method. First, a photosensitive etching resist was applied to both surfaces of the stainless-steel plate. These resist layers were subjected to pattern exposure and development to form resist patterns on both surfaces of the stainless-steel plate. Then, these resist patterns were used as an etching mask to etch the stainless steel. Ferric chloride solution was used as the etching liquid. Then, the resist patterns were stripped to obtain a metal plate 20 having a slit 27. Each substrate 21 had a square surface of 0.8 mm × 0.8 mm.

### (B) Formation of protective layer

Then, a continuous carbon film with a thickness of 3 µm was formed as the protective layer 22 shown in Figs. 33A to 33C by plating on the entire surface of the metal plate 20. Specifically, the protective layer 22 was formed by immersing the metal plate 20 in a molten salt (LiCl-KCl-CaCl₂: 500°C) containing calcium carbide, followed by anode polarization.

### (C) Resin molding

Then, the metal plate 20 formed with the protective layer 22 was placed in the cavity of a mold, not shown. The metal plate 20 was brought into pressure contact with the mold so that the portion of the protective layer 22 positioned on the main surface of each substrate 21 adhered to the inner wall of the mold. Thus, the above portion was ensured not to be covered with resin. Then, a resin was injected into the mold, followed by curing. Then, the mold was disassembled to take out the molded article including the metal plate 20 from the mold. Thus, the support 3 shown in Figs. 34A to 34C and 35 was obtained.

### (D) Separation of electrodes and formation of electrode unit

Then, the portions of the protective layer 22 located on the main surfaces of the respective substrates 21 were immersed in a 0.001 M hydrogen hexachloroplatinate solution, and a negative voltage was applied thereto for 10 seconds (cathode polarization). Thus, a very small amount of platinum was permitted to adhere to the portions of the protective layer 22 located on the main surfaces of the respective substrates 21. Then, as shown in Fig. 36, a laser beam was irradiated on the back surfaces of the linear parts 25b and 25c so as to cut each of the linear parts 25b at positions Pb, and cut each of the linear parts 25c at positions Pc.

Then, the support layer 34 was cut into a lattice shape so that the cutting lines extending in the transverse direction pass through the positions Pb, and the cutting lines extending in the longitudinal direction pass through the positions Pc. Thus, first composites each containing the electrode 2a and part of the support layer 34, second composites each containing the electrode 2b and another part of the support layer 34, and third composites each having the same configuration as the first or second composite were obtained.

Then, a first composite, a second composite and a third composite were bonded to a separately prepared frame made of a resin, and a silver / silver chloride reference electrode was arranged in the vicinity thereof.

### (E) Formation of connection portions

A carbon paste was supplied to the back surfaces of the electrodes in the first to third composites by a dispenser to form connection bumps.

### (F) Preparation of electronic component

The component shown in Fig. 19 was prepared. Specifically, a semiconductor chip including a signal processing unit 4a, a semiconductor chip including an output unit 4c and a semiconductor chip including a power supply unit 4d were used. Also, a printed wiring board PWB on which these chips were mounted was used.

### (G) Connection of components

The semiconductor chip including the signal processing unit 4a was die-bonded to the printed wiring board PWB, and a terminal of the semiconductor chip was wire-bonded to a terminal on the printed wiring board PWB. The electrode with the structure obtained through the process (E) was electrically connected to the terminal of the semiconductor chip via a lead wire. Other semiconductor chips were flip chip-bonded or wire-bonded to the respective terminals on the printed wiring board PWB. Thus, the electronic component 4 shown in Fig. 19 was obtained.

Then, an interface 41 was electrically connected to the signal processing unit 4a to input data thereto. Further, an interface 42 was electrically connected to the output unit 4c to externally transmit data therefrom. Furthermore, an external power supply 5 was electrically connected to the power supply unit 4d. In this way, the biosensor 1 shown in Fig. 19 was completed.

### (H) Measurements

In a living body, cellular activities produce nitrogen-containing oxidation substances (RNS: reactive nitrogen species) and oxygen-containing oxidation substances (ROS: reactive oxygen species). These oxidation substances change into different substances over time, but if accumulated and the concentration becomes high, these substances are considered to cause damage to cells and be the cause of various diseases. Examples of RNS include ONOO⁻, NO₂⁻, NO^{•}, and NO⁻. As ROS, O₂^{•-}, H₂O₂, OH^{•} and the like are known. Since a plurality of such species simultaneously exist in a living body, it is highly significant to find a means for simultaneously measuring these species. Therefore, as described below, components were simultaneously detected by the biosensor 1, although only by simulation, for a solution containing a plurality of RNS and ROS.

First, H₂O₂ and NO₂⁻ were added to phosphate buffered saline (pH 7.2) to prepare a first solution containing a 1 mM concentration of H₂O₂ and a 1 mM concentration of NO₂⁻, a second solution containing a 2 mM concentration of H₂O₂ and a 1 mM concentration of NO₂⁻, and a third solution containing a 2 mM concentration of H₂O₂ and a 2 mM concentration of NO₂⁻.

Then, each of these solutions were used as the sample 10 shown in Fig. 10 and analyzed by the biosensor 1. Specifically, the amount of each sample 10 was 1 mL. A voltage of 0.4 V was applied across the reference electrode and the electrode 2a, and a voltage of 0.9 V was applied across the reference electrode and the electrode 2b. The electrode 2c was used as a common counter electrode. The current flowing through the electrodes 2a and 2b was measured 60 seconds after start of the application of the voltage. Part of the measurement conditions are shown in the following Table 1. The results of the measurements are shown in Table 2 below.

**[Table 1]**

| | Electrode 2a | Electrode 2b |
|---|---|---|
| Potential (vs. Ag-AgCl; V) | 0.4 | 0.9 |
| Measuring object | H₂O₂ | H₂O₂ + NO₂ |

**[Table 2]**

| Sample | Concentration (mM) | | Current (µA) | |
|---|---|---|---|---|
| | H₂O₂ | NO₂ | Electrode 2a | Electrode 2b |
| First solution | 1 | 1 | 2.7 | 4.9 |
| Second solution | 2 | 1 | 5.5 | 8.1 |
| Third solution | 1 | 2 | 2.4 | 7.6 |

According to a reference document (Y. Li et al., Electrochimica Acta, vol. 144, 111-118 (2014)), the "measuring objects" indicated in Table 1 are detected at the potentials set therein. At 0.4 V, only H₂O₂ is detected, at 0.9 V, H₂O₂ and NO₂⁻ are detected together, and the current is substantially proportional to the amount of these substances present. As will be understood from the measurements shown in Table 2, the biosensor 1 is capable of simultaneously measuring a plurality of oxidation species (herein, H₂O₂ and NO₂⁻).

### <Example 3>

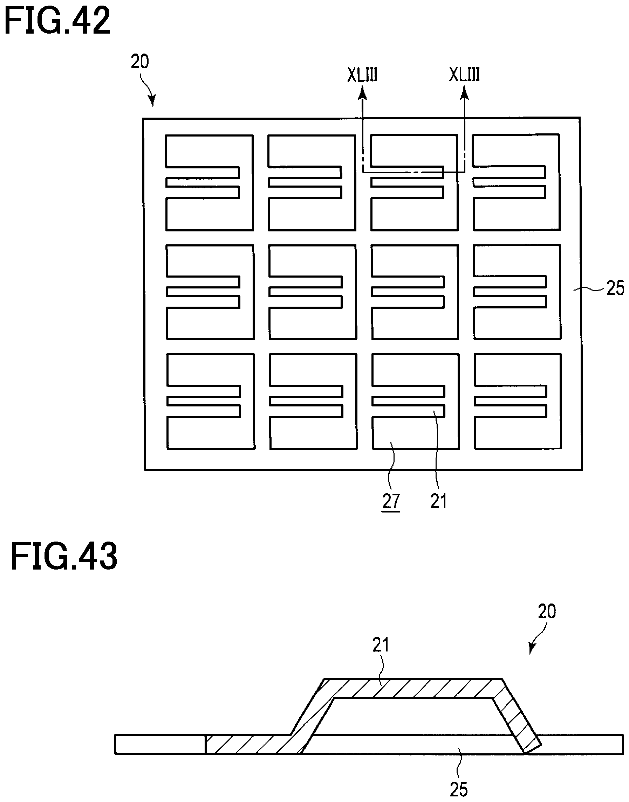

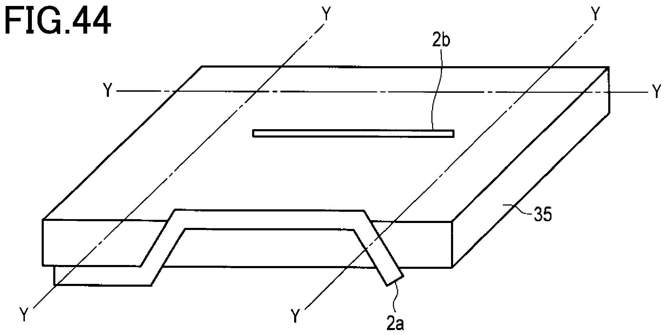

Fig. 42 is a schematic plan view illustrating a process in the manufacture of a biosensor according to Example 3 of the present invention. Fig. 43 is a cross-sectional view taken along the line XLIII-XLIII of the structure shown in Fig. 42. Fig. 44 is a schematic perspective view illustrating a process in the manufacture of the biosensor according to Example 3 of the present invention. In the present example, a biosensor was manufactured by the method described below, and measurements were performed using the biosensor.

### (A) Processing of materials

Stainless steel (SUS304) with a thickness of 0.3 mm was processed to obtain the metal plate 20 shown in Figs. 42 and 42. Specifically, the stainless steel was processed by the following method. First, a photosensitive etching resist was applied to both surfaces of the stainless-steel plate. These resist layers were subjected to pattern exposure and development to form resist patterns on both surfaces of the stainless-steel plate. Then, these resist patterns were used as an etching mask to etch the stainless steel. Ferric chloride solution was used as the etching liquid. Then, the resist patterns were stripped to obtain a metal plate 20 having a slit 27. Then, the metal plate 20 was subjected to bending to partially uplift each of the substrates 21 with respect to the metal plate 20. Herein, each substrate 21 had a length of 8 mm, and was uplifted by 2 mm with respect to the support part 25.

### (B) Formation of protective layer

Then, a continuous carbon film with a thickness of 3 µm was formed, as a protective layer 22 shown such as in Figs. 33A to 33C, by plating on an overall surface of the metal plate 20. Specifically, the protective layer 22 was formed by immersing the metal plate 20 in a molten salt containing calcium carbide (LiCl-KCl-CaCl₂: 500°C), followed by anode polarization.

### (C) Resin molding

Then, the metal plate 20 on which the protective layer 22 was formed was placed in the cavity of a mold, not shown. Then, a resin was injected into the mold, followed by curing. Thus, the structure shown in Fig. 44 was obtained. The resin was molded so that the portions of each substrate 21 used as the electrodes 2a and 2b, and the portions thereof connected to the lead wires, which will be described hereinafter, respectively project from the front and back surfaces of the resin support 35.

### (D) Temporary arrangement of electrodes and connection of lead wires

Then, the structure shown in Fig. 44 was cut along the lines Y-Y by a dicing apparatus to obtain the structure having a main surface of 5 mm × 5 mm. Thus, the electrodes 2a and 2b were separated from the support part 25.

Then, an end of a lead wire 6a and an end of a lead wire 6b shown in Fig. 19 were respectively bonded to the electrodes 2a and 2b. Copper wires was used as the lead wires 6a and 6b. The lead wires 6a and 6b were bonded to the portions of the substrate 21 projected from the back surface of the support 35 using a conductive paste.

### (E) Preparation of electronic component

The printed wiring board PWB shown in Fig. 19 was prepared. Semiconductor modules as a signal processing unit 4a and an output unit 4c, and a semiconductor module as a power supply unit 4d were mounted on the printed wiring board PWB. Thus, an electronic component 4 was obtained.

### (F) Connection of components

The signal processing unit 4a of the electronic component 4 was electrically connected with an interface 41 for inputting data to the signal processing unit 4a, and with the lead wires 6a, 6b and 6c. Further, the output unit 4c of the electronic component 4 was electrically connected with an interface 42 for externally transferring data from the output unit 4c. Furthermore, the power supply unit 4d of the electronic component 4 was electrically connected with an external power supply 5. In this way, the biosensor 1 shown in Fig. 19 was completed.

### (G) Measurements

10 µL of blood was mixed with 10 µL of physiological saline containing glucose oxidase and ferricyanide ions each at a concentration of 0.01 mol/L, and this mixture was dripped on a preparation. 1 minute after start of mixing, the electrode unit 2 was pressed against the mixture from above, and a voltage of 0.5 V was applied across the electrodes 2a and 2b. Then, the current flowing across the electrodes 2a and 2b was measured 10 seconds after start of the application of the voltage. The current resulting from this measurement was 1 to 10 µA at a blood glucose concentration in the range of 10 to 500 mg/dL, which meant that current linearity was high with respect to the blood glucose concentration. This measurement method is comparatively common as a detection method of blood glucose concentration. Through the above examination, it was found that accurate measurements can be conducted using the aforementioned biosensor 1.

## Claims

1. A biosensor, comprising two or more electrodes and a support supporting the two or more electrodes, wherein:
one or more of the electrodes each include a substrate having a metal surface, and a protective layer covering at least part of the surface; and
the protective layer is a continuous film made of carbon and having a thickness of 1 µm or more.

2. The biosensor according to claim 1, wherein the protective layer covers at least an entire region of the surface with which a measuring object can be in contact.

3. The biosensor according to claim 1 or 2, further comprising a lead wire having an end bonded to the surface, wherein the protective layer covers an entirety of the surface except for a joint between the surface and the lead wire.

4. The biosensor according to any one of claims 1 to 3, wherein the substrate has a curved or bent shape, and the protective layer covers at least a region of the surface corresponding to a curved or bent portion of the substrate.

5. The biosensor according to any one of claims 1 to 4, wherein the substrate includes a first main surface and a second main surface parallel to each other, and an edge face extending along edges of the main surfaces, and the protective layer covers the entirety of the first main surface, at least part of the second main surface, and at least part of the edge face.

6. The biosensor according to claim 5, wherein the support supports the two or more electrodes so that the electrodes are inclined against each other.

7. The biosensor according to any one of claims 1 to 4, wherein the substrate has a coil shape or a leaf-spring shape.

8. The biosensor according to any one of claims 1 to 7, further comprising a processing unit electrically connected to the two or more electrodes, and generating information relating to a measuring object from current flowing across the electrodes or voltage across the electrodes.

9. The biosensor according to claim 8, further comprising an output unit outputting the information to the outside of the biosensor.

10. A manufacturing method for a biosensor comprising:
a step of obtaining an electrode by forming a carbon protective layer using plating on a substrate having a metal surface so as to cover at least part of the surface and to have a thickness of at least 1 µm; and
a step of allowing a support to support two or more electrodes including the electrode.

11. The manufacturing method for a biosensor according to claim 10, further comprising a step of deforming the substrate prior to the step of obtaining an electrode.

12. The manufacturing method for a biosensor according to claim 10 or 11, further comprising:
a step of obtaining a structure including the substrate and a support connected to the substrate, by removing part of a plate having the metal surface; and
a step of cutting off the substrate from the support after the step of obtaining an electrode.

13. The manufacturing method for a biosensor according to claim 12, further comprising bonding a lead wire to a portion of the substrate exposed by being cut off from the support.

14. The manufacturing method for a biosensor according to claim 12, further comprising bonding a lead wire to an exposed portion of the surface exposed by removing part of the protective layer.
